(19) Europäisches Patentamt
European Patent Office
Office européen des brevets

(11) EP 4 710 958 A1

(12) EUROPEAN PATENT APPLICATION
published in accordance with Art. 153(4) EPC

(43) Date of publication:
**18.03.2026 Bulletin 2026/12**

(21) Application number: **24807018.7**

(22) Date of filing: **26.04.2024**

(51) International Patent Classification (IPC):
***A61L 27/34*** (2006.01) ***A61L 15/24*** (2006.01)
***A61L 15/26*** (2006.01) ***A61L 27/16*** (2006.01)
***A61L 27/18*** (2006.01) ***A61L 27/52*** (2006.01)
***A61L 31/06*** (2006.01) ***A61L 31/10*** (2006.01)
***A61L 31/14*** (2006.01) ***A61L 33/06*** (2006.01)

(52) Cooperative Patent Classification (CPC):
**A61L 15/24; A61L 15/26; A61L 27/16; A61L 27/18; A61L 27/34; A61L 27/52; A61L 31/06; A61L 31/10; A61L 31/14; A61L 33/06**

(86) International application number:
**PCT/JP2024/016431**

(87) International publication number:
**WO 2024/237064 (21.11.2024 Gazette 2024/47)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **12.05.2023 JP 2023079081**

(71) Applicant: **Toray Industries, Inc.**
**Tokyo 103-8666 (JP)**

(72) Inventor: **KITAGAWA Rumiko**
**Otsu-shi, Shiga 520-8558 (JP)**

(74) Representative: **Hoefer & Partner Patentanwälte mbB**
**Pilgersheimer Straße 20**
**81543 München (DE)**

# (54) COATED DEVICE AND PRODUCTION METHOD FOR SAME

(57) The purpose of the present invention is to provide: a coated device that has been given lipid and protein adhesion-suppressing characteristics in addition to substantial hydrophilicity and slipperiness; and a simple production method for the coated device. The present invention provides a coated device that comprises a device and a hydrophilic polymer that coats the surface of the device. The hydrophilic polymer includes a hydrophilic polymer A. The hydrophilic polymer A includes, as monomer units, a compound a1 that has a specific structure, a compound a2 that has an amide group, and a compound a3 that has a plurality of hydroxyl groups. The copolymerization proportions of the compound a1, the compound a2, and the compound a3 are 1-96 mass% for the compound a1, 1-96 mass% for the compound a2, and 8-61 mass% for the compound a3.



Processed by Luminess, 75001 PARIS (FR)

## Description

TECHNICAL FIELD

The present invention relates to a coated device and a method for the production thereof.

BACKGROUND ART

Conventionally, devices incorporating soft materials such as silicone rubber or hydrogel and devices incorporating hard materials such as metal and glass have been used in a wide range of applications across various fields.

For example, in the case of medical devices to be inserted into bodies or attached to biological surfaces, it is important to subject these medical devices to surface modification to ensure improved biocompatibility. Surface modification is expected to serve to realize better user comfort, reduced discomfort, symptom improvement, and the like if it works to impart properties such as hydrophilicity, lubricity, and lipid adhesion resistance, in addition to enhanced biocompatibility, to these medical devices.

For example, a known method for surface modification of a medical device is to immerse the medical device in a solution of pH 6 to 9 containing a polymer produced from a compound having an amide group such as N,N-dimethylacrylamide or vinylpyrrolidone at room temperature, followed by heating the medical device after the immersion as required (Patent documents 1 to 3).

PRIOR ART DOCUMENTS

PATENT DOCUMENTS

Patent document 1: International Publication WO 2017/018425
Patent document 2: International Publication WO 2015/119256
Patent document 3: Japanese Patent No. 5154231

SUMMARY OF INVENTION

PROBLEMS TO BE SOLVED BY THE INVENTION

However, the inventions disclosed in Patent documents 1 to 3 do not work sufficiently in imparting hydrophilicity, lubricity, or the like to the surfaces of medical devices, and do not serve either to impart lipid adhesion resistance to medical devices.

In view of these issues, the main object of the present invention is to provide a coated device that possesses lipid and protein adhesion resistance in addition to sufficient hydrophilicity and lubricity, and also provide a simple method for the production thereof.

MEANS OF SOLVING THE PROBLEMS

To achieve the above object, the present invention provides a coated device including a device and a hydrophilic polymer that coats the surface of the device, wherein the hydrophilic polymer includes a hydrophilic polymer A, the hydrophilic polymer A containing, as monomer units, a compound a1 as represented by the general formula (I) given below, a compound a2 having an amide group, and a compound a3 having a plurality of hydroxyl groups, the polymerization ratio among the compound a1, compound a2, and compound a3 being such that the compound a1 accounts for 1 to 96 mass%, the compound a2 for 1 to 96 mass%, and the compound a3 for 8 to 61 mass%.

[Chemical compound 1]

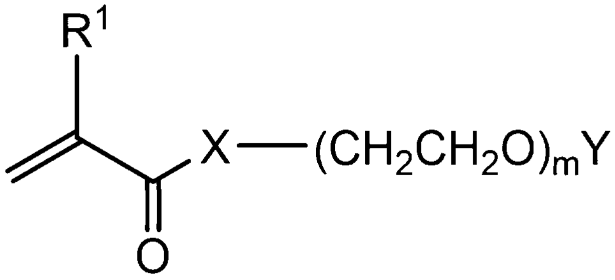

・・・(I)

In the general formula (I), $R^1$ represents a hydrogen atom or a methyl group; X represents an oxygen atom or $NR^2$; $R^2$ represents a hydrogen atom or an alkyl group; m represents an integer of 1 to 30; and Y represents a hydrogen atom or an alkyl group.

The present invention also provides a method for producing a coated device, including a contact step (A) in which a device is placed in a container, followed by bringing the device into contact with a solution a of a hydrophilic polymer A and a heating step (C) in which the container is heated, wherein the hydrophilic polymer A contains, as monomer units, a compound a1 as represented by the general formula (I) given above, a compound a2 having an amide group, and a compound a3 having a plurality of hydroxyl groups, the polymerization ratio among the compound a1, compound a2, and compound a3 being such that the compound a1 accounts for 1 to 96 mass%, the compound a2 for 1 to 96 mass%, and the compound a3 for 8 to 61 mass%, and the solution a after the heating step having a pH in the range of 6.1 to 8.0.

ADVANTAGEOUS EFFECTS OF THE INVENTION

According to the present invention, it is possible to provide a coated device having lipid and protein adhesion resistance in addition to sufficiently high hydrophilicity and lubricity required particularly when the device is a medical device. Furthermore, the production method according to the present invention serves to produce such a coated device through a simple process.

DESCRIPTION OF PREFERRED EMBODIMENTS

The coated device according to the present invention includes a device and a hydrophilic polymer that coats the surface of the device. Here, the hydrophilic polymer is characterized by including a hydrophilic polymer A, and the hydrophilic polymer A contains, as monomer units, a compound a1 as represented by the general formula (I) given below, a compound a2 having an amide group, and a compound a3 having a plurality of hydroxyl groups, wherein the polymerization ratio among the compound a1, compound a2, and compound a3 is such that the compound a1 accounts for 1 to 96 mass%, the compound a2 for 1 to 96 mass%, and the compound a3 for 8 to 61 mass%.

[Chemical compound 2]

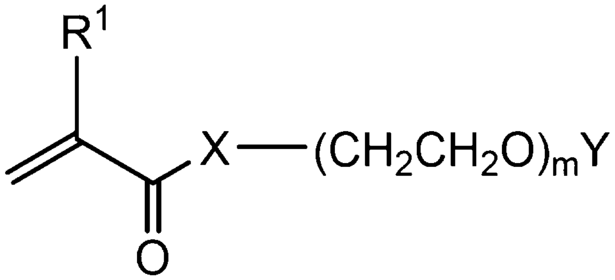

・・・(I)

[In the general formula (I), $R^1$ represents a hydrogen atom or a methyl group; X represents an oxygen atom or $NR^2$; $R^2$ represents a hydrogen atom or an alkyl group; m represents an integer of 1 to 30; and Y represents a hydrogen atom or an alkyl group.]

Examples of devices to be incorporated in the coated device according to the present invention include ophthalmic lenses, skin covering materials, wound dressing materials, skin protective materials, skin drug carriers, infusion tubes, gas transport tubes, liquid drainage tubes, blood circuits, coating tubes, catheters, stents, sheaths,

biosensor chips, artificial heart and lung components, and endoscope covering materials. Here, examples of ophthalmic lenses include contact lenses, intraocular lenses, artificial corneas, corneal inlays, corneal onlays, and spectacle lenses.

The material used to form such a device may be a water-containing material or a low-water-content material. Examples of water-containing materials include hydrogel and silicone hydrogel. In the case where the device is a contact lens, the use of silicone hydrogel is preferable because it can form a durable surface having high lipid and protein adhesion resistance, high hydrophilicity, and high lubricity, and easily maintain prolonged effect during long wear. Examples of low-water-content materials include low-water-content soft materials and low-water-content hard materials. Here, the term "low-water-content materials" refers to materials having a water content of 10 mass% or less.

Hereinafter, the names of hydrogel materials or silicone hydrogel materials will occasionally be shown using United States Adopted Names (USAN). According to USAN, a letter such as A, B, or C may be appended to indicate a variant of a material. In the present Description, all variants are intended when no such suffix is appended. For example, when simply stated as "ocufilcon", it represents all ocufilcon variants including "ocufilcon A", "ocufilcon B", "ocufilcon C", "ocufilcon D", "ocufilcon E", and "ocufilcon F."

Examples of hydrogel materials include tefilcon, tetrafilcon, hefilcon, mafilcon, polymacon, hioxifilcon, alfafilcon, omafilcon, hixoifilcon, nelfilcon, nesofilcon, hilafilcon, acofilcon, deltafilcon, etafilcon, focofilcon, ocufilcon, phemfilcon, methafilcon, and vilfilcon.

In the case where the device is a contact lens made of a hydrogel material, it is classified into any of Groups 1 to 4 according to the classification for contact lenses specified by the U.S. Food and Drug Administration (FDA). It preferably falls under Group 2 or Group 4, which requires high hydrophilicity, and more preferably falls under Group 4.

Examples of Group 1 nonionic hydrogel materials with water contents of less than 50 mass% include tefilcon, tetrafilcon, helfilcon, mafilcon, polymacon, and hioxifilcon.

Examples of Group 2 nonionic hydrogel materials with water contents of 50 mass% or more include alfafilcon, omafilcon, hixoifilcon, nelfilcon, nesofilcon, hilafilcon, and acofilcon. Among these, omafilcon, hixoifilcon, nelfilcon, and nesofilcon are preferable due to their high hydrophilicity, of which omafilcon and hixoifilcon are more preferable, with omafilcon being still more preferable.

Examples of Group 3 ionic hydrogel materials with water contents of less than 50 mass% include deltafilcon.

Examples of Group 4 ionic hydrogel materials with water contents of 50 mass% or more include etafilcon, focofilcon, ocufilcon, phemfilcon, methafilcon, and vilfilcon. Among these, etafilcon, focofilcon, ocufilcon, and phemfilcon are preferable due to their high hydrophilicity, of which etafilcon and ocufilcon are more preferable, with etafilcon being still more preferable.

In the case where the device is a contact lens made of a silicone hydrogel material, it preferably falls under Group 5 according to the contact lens classification specified by the U.S. Food and Drug Administration (FDA).

Preferable silicone hydrogel materials classified into Group 5 include polymers having silicon atoms in the main chain and/or side chain and possessing hydrophilicity, and examples thereof include copolymers formed from monomers containing siloxane bonds and hydrophilic monomers. Such copolymers include, for example, lotrafilcon, galyfilcon, narafilcon, senofilcon, comfilcon, enfilcon, balafilcon, efrofilcon, fanfilcon, somofilcon, samfilcon, olifilcon, asmofilcon, formofilcon, stenfilcon, abafilcon, mangofilcon, riofilcon, sifilcon, larafilcon, kalifilcon, and delefilcon. Among these, lotrafilcon, galyfilcon, narafilcon, senofilcon, comfilcon, enfilcon, stenfilcon, somofilcon, delefilcon, balafilcon, kalifilcon, and samfilcon are preferable because of having high hydrophilicity and antifouling property in addition to lipid adhesion resistance, of which lotrafilcon, narafilcon, senofilcon, comfilcon, kalifilcon, and enfilcon are more preferable, with narafilcon, senofilcon, kalifilcon, and comfilcon being still more preferable.

As a low-water-content soft material or a low-water-content hard material, the use of a material containing silicon atoms, which exhibit high oxygen permeability to ensure sufficient oxygen supply to corneal, is preferable in the case where the device is a contact lens.

As a low-water-content hard material, it is preferable to select a low-water-content hard material that falls under the category of contact lens materials specified by the U.S. Food and Drug Administration (FDA).

As such a low-water-content hard material, it is preferable to use a polymer having silicon atoms in the main and/or side chain in the form of siloxane bonds etc., more preferably a homopolymer formed of a tris(trimethylsiloxy) silylpropyl methacrylate, which is high in oxygen permeability, a polydimethylsiloxane, which has double bonds at both chain ends, a silicone-containing acrylate, a silicone-containing methacrylate, or a copolymer thereof with other monomers.

Specifically, it is preferable to select a low-water-content hard material from the group consisting of neofocon, pasifocon, telefocon, silafocon, paflufocon, petrafocon, and fluorofocon. Among these, neofocon, pasifocon, telefocon, and silafocon are more preferable because they exhibit high lipid adhesion resistance and good antifouling property, of which neofocon, pasifocon, and telefocon are still more preferable, with neofocon being particularly preferable.

In the case where the device is not a contact lens, examples of preferable low-water-content hard materials include polyethylene, polypropylene, polysulfone, polyetherimide, polystyrene, polymethyl methacrylate, polyamide, polyester, epoxy resin, polyurethane, and polyvinyl chloride. Among these, polysulfone, polystyrene, polymethyl methacrylate, polyurethane, and polyamide are more preferable due to their high lipid adhesion resistance and good antifouling property, with polymethyl methacrylate being still more preferable.

Examples of low-water-content soft materials include the materials disclosed in International Publication No. WO2013/024799, which has a water content of 10 mass% or less, an elastic modulus of 100 to 2,000 kPa, and a tensile elongation of 50 to 3,000%, as well as elastofilcon.

The present invention serves to impart a moderate degree of lipid and protein adhesion resistance to the surface of a device regardless of whether the device is of a water-containing material or a low-water-content material, but it is preferable for the device to have a water content of 0.0001 mass% or more, more preferably 0.001 mass% or more. On the other hand, the water content of the device is preferably 80 mass% or less, more preferably 70 mass% or less, and still more preferably 60 mass% or less.

In the case where the device is a contact lens, the water content of the device is preferably 15 mass% or more, and more preferably 20 mass% or more, in order to easily ensure proper movement of the lens on the eye.

The aforementioned hydrophilic polymer A has hydrophilicity. Here, the expression "to have hydrophilicity" means that the polymer has a solubility of 0.0001 part by mass or more in 100 parts by mass of water or in a mixed solution of 100 parts by mass of water and 100 parts by mass of tert-butanol at room temperature (20°C to 23°C). It preferably has a solubility of 0.01 part by mass or more, more preferably has a solubility of 0.1 part by mass or more, and still more preferably has a solubility of 1 part by mass or more.

The aforementioned hydrophilic polymer A contains, as monomer units, a compound a1 as represented by the general formula (I) given below, a compound a2 having an amide group, and a compound a3 having a plurality of hydroxyl groups, wherein the polymerization ratio among the compound a1, compound a2, and compound a3 is such that the compound a1 accounts for 1 to 96 mass%, the compound a2 for 1 to 96 mass%, and the compound a3 for 8 to 61 mass%. Here, the term "the polymerization ratio among the compound a1, compound a2, and compound a3" refers to the ratio among the mass contents of the compound a1, compound a2, and compound a3 that are represented relative to the total mass of the compound a1, compound a2, and compound a3, which accounts for 100%.

The compound a1, compound a2, and compound a3 may each be a single monomer or a mixture of a plurality of monomers having different structures.

From the viewpoint of high polymerizability, the compound a1, compound a2, and compound a3 are each preferably a monomer having an allyl group, a vinyl group, or a (meth)acryloyl group, and particularly preferably a monomer having a (meth)acryloyl group.

[Chemical compound 3]

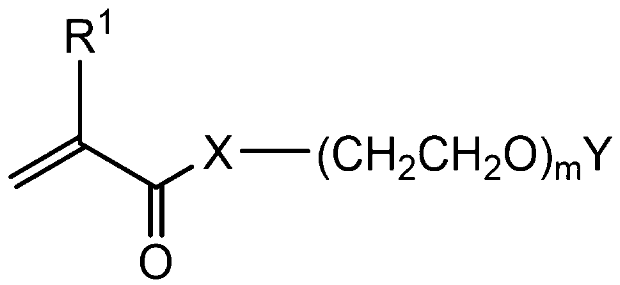

・・・( I )

In the general formula (I), $R^1$ represents a hydrogen atom or a methyl group; X represents an oxygen atom or $NR^2$; $R^2$ represents a hydrogen atom or an alkyl group; m represents an integer of 1 to 30; and Y represents a hydrogen atom or an alkyl group.

When $R^2$ is an alkyl group, it may be either linear or branched, but it is preferably an alkyl group having 1 to 10 carbon atoms. Examples of $R^2$ include the methyl group, ethyl group, propyl group, 2-propyl group, butyl group, 2-butyl group, tert-butyl group, pentyl group, 2-pentyl group, 3-pentyl group, hexyl group, heptyl group, and octyl group.

From the viewpoint of ensuring an appropriate degree of hydrophilicity and ease of polymerization, m is preferably in the range of 1 to 30. The lower limit of m is more preferably 1, still more preferably 2, and particularly preferably 4. The upper limit of m is more preferably 25, still more preferably 13, and particularly preferably 9.

From the viewpoint of ease of coat formation, Y is preferably an alkyl group having 1 to 5 carbon atoms, and it is more preferably a methyl group, ethyl group, propyl group, isopropyl group, or butyl group, and particularly preferably a methyl group or ethyl group, and most preferably a methyl group.

From the viewpoint of ease of polymerization, preferable examples of the compound a2 having an amide group include compounds having an acrylamide group or a methacrylamide group, and N-vinylcarboxylic acid amide (which may have a cyclic structure).

Specific examples include N-vinylpyrrolidone, N-vinylcaprolactam, N-vinylacetamide, N-methyl-N-vinylacetamide, N-vinylformamide, N,N-dimethylacrylamide, N-isopropylacrylamide, N-methylacrylamide, N-ethylacrylamide, N-butylacrylamide, N-tert-butylacrylamide, N-hydroxymethylacrylamide, N-methoxymethylacrylamide, N-ethoxymethylacrylamide, N-propoxymethylacrylamide, N-isopropoxymethylacrylamide, N-(2-hydroxyethyl)acrylamide, N-butoxymethylacrylamide, N-isobutoxymethylacrylamide, N-hydroxymethylmethacrylamide, N-methoxymethylmethacrylamide, N-ethoxymethylmethacrylamide, N-propoxymethylmethacrylamide, N-butoxymethylmethacrylamide, N-isobutoxymethylmethacrylamide, acryloylmorpholine, and acrylamide. In order to ensure increased lubricity, N-vinylpyrrolidone, N-isopropylacrylamide, and N,N-dimethylacrylamide are preferable, of which N-isopropylacrylamide and N,N-dimethylacrylamide are more preferable, with N,N-dimethylacrylamide being still more preferable.

Examples of the compound a3 having a plurality of hydroxyl groups include glycerol acrylate, glycerol methacrylate, divalent phenyl (meth)acrylate, trivalent phenyl (meth)acrylate, compounds as represented by the structural formula (a) given below, and compounds as represented by the structural formula (b) given below. The expression "having a plurality of hydroxyl groups" means possessing two or more hydroxyl groups (OH groups). It should be noted that the OH group present in a carboxyl group (COOH group) and the hydroxyl groups directly bonded to atoms other than carbon are excluded.

[Chemical compound 4]

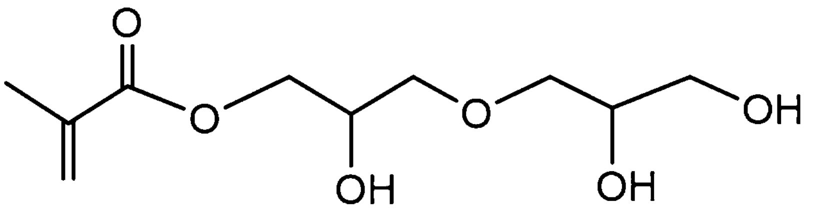

• • • (a)

[Chemical compound 5]

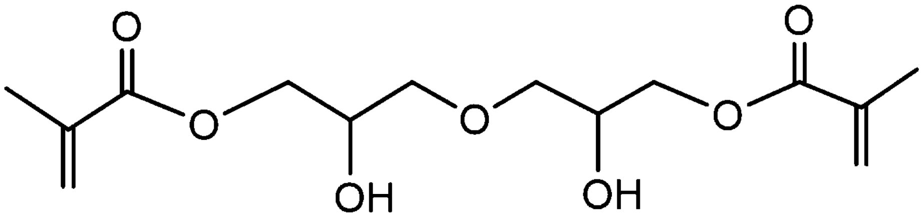

• • • (b)

To ensure increased adsorption to the device, it is preferable, from the viewpoint of ensuring an appropriate degree of hydrophilicity and ease of polymerization, to adopt a chain compound having a plurality of hydroxyl groups such as glycerol acrylate, glycerol methacrylate, 2-methacryloyloxyethyl acid phosphate, a compound as represented by the structural formula (a) given above, and a compound as represented by the structural formula (b) given above, of which the use of glycerol acrylate or glycerol methacrylate is preferable. Here, the term "chain compound" refers to a compound that has no cyclic structures. A chain compound may also be referred to as acyclic compound in some cases.

The hydrophilic polymer A may also include, as monomer units, one or more other compounds, which are introduced through copolymerization, in addition to a compound a1 as represented by the general formula (I), a compound a2 having an amide group, and a compound a3 having a plurality of hydroxyl groups. Examples of such other compounds include N-(4-hydroxyphenyl)maleimide, hydroxystyrene, vinylalcohol (using vinyl carboxylic acid esters as precursors), hydroxyethyl (meth)acrylate, hydroxypropyl (meth)acrylate, hydroxybutyl (meth)acrylate, caprolactone modified hydroxyethyl (meth)acrylate, (methacrylic acid, vinylbenzoic acid, thiophene-3-acetic acid, 4-styrenesulfonic acid, vinylsulfonic acid, and 2-acrylamido-2-methylpropanesulfonic acid, as well as salts thereof. To enhance the antifouling property against body fluids, hydroxyethyl (meth)acrylate, vinyl alcohol, and (meth)acrylic acid are preferable, of which hydroxyethyl (meth) acrylate is more preferable. Such other compounds may also include those exhibiting functions such as hydrophilicity, antimicrobial activity, antifouling property, and pharmacological efficacy.

The inclusion of the compound a1 represented by the general formula (I) as a monomer unit in the hydrophilic polymer A serves to facilitate easy dissolution of the hydrophilic polymer A in water, development of hydrophilic functionality, and formation of a surface that exhibits lipid and protein adhesion resistance as well as hydrophilicity.

The inclusion of the compound a2 having an amide group as a monomer in the hydrophilic polymer A serves to develop an appropriate degree of viscosity, enabling the formation of a surface with not only hydrophilicity but also lubricity,

as the hydrophilic polymer A is dissolved in water.

The inclusion of the compound a3 having a plurality of hydroxyl groups as a monomer in the hydrophilic polymer A serves to enable easy adsorption of the hydrophilic polymer A to the surface of the device to form a durable hydrophilic surface.

In order to facilitate the development of properties such as lipid and protein adhesion resistance as well as durable hydrophilicity, the copolymer proportion of the compound a1 is in the range of 1 to 96 mass%. Actually, the proportion of the compound a1 should be 1 mass% or more, and it is preferably 5 mass% or more, and more preferably 10 mass% or more, relative to the total mass of all monomers present in the hydrophilic polymer A, which accounts for 100 mass%. In addition, from the viewpoint of ease of polymerization, the proportion of the compound a1 should be 96 mass% or less, and it is preferably 70 mass% or less, more preferably 60 mass% or less, and still more preferably 40 mass% or less.

To facilitate the development of properties such as durable hydrophilicity and lubricity, the proportion of the compound a2 is in the range of 1% to 96% relative to the total mass of all monomers present in the hydrophilic polymer A, which accounts for 100 mass%. The proportion of the compound a2 should be 1 mass% or more, and it is preferably 10 mass% or more, more preferably 30 mass% or more, and still more preferably 40 mass% or more. On the other hand, the proportion of the compound a2 should be 90 mass% or less, and it is preferably 90 mass% or less, more preferably 80 mass% or less, and still more preferably 70 mass% or less.

To facilitate easy polymerization and development of adsorptivity to the surface of the device, the proportion of the compound a3 is in the range of 8% to 61% relative to the total mass of all monomers present in the hydrophilic polymer A, which accounts for 100 mass%. The proportion of the compound a3 should be 8 mass% or more, and it is preferably 9 mass% or more, more preferably 10 mass% or more, and still more preferably 20 mass% or more. On the other hand, the proportion of the compound a3 should be 60 mass% or less, and it is preferably 55 mass% or less, more preferably 50 mass% or less, and still more preferably 45 mass% or less.

If another compound is copolymerized as a fourth monomer component in addition to the compound a1 represented by the general formula (I), compound a2 having an amide group, and compound a3 having a plurality of hydroxyl groups, its proportion is preferably 2 mass% or more, more preferably 5 mass% or more, and still more preferably 10 mass% or more, from the viewpoint of ease of polymerization. Meanwhile, the copolymer proportion of the fourth monomer component is preferably 90 mass% or less, more preferably 60 mass% or less, and still more preferably 50 mass% or less.

If the copolymer proportions of the compound a1 represented by the general formula (I), the compound a2 having an amide group, the compound a3 having a plurality of hydroxyl groups, and the fourth monomer component are in the aforementioned ranges, it serves to enable the development of properties such as lubricity and antifouling property against body fluids.

In order to suppress deformation of the device while ensuring improved durability in addition to high hydrophilicity and lubricity, the detection depth of the hydrophilic polymer A is preferably 10.00 μm or less from the outermost surface of the coated device. It is more preferably 4.00 to 5.00 μm, still more preferably 3.00 to 4.00 μm, particularly preferably 2.00 to 3.00 μm, and most preferably 0.05 to 2.00 μm.

The detection depth of the hydrophilic polymer A can be determined by analyzing the surface of the coated device by means of time-of-flight secondary ion mass spectrometry (hereinafter referred to as TOF-SIMS).

The detection depth of the hydrophilic polymer A refers to the distance (depth) from the surface to the point where the detection intensity of the ion species corresponding to the hydrophilic polymer A detected when measured in the depth direction from the surface of the coated device is equal to the detection intensity (background) of the ion species corresponding to the hydrophilic polymer A detected from the device itself (uncoated). In the case where the device is not coated with a hydrophilic polymer containing the hydrophilic polymer A, the aforementioned detection depth of the hydrophilic polymer is 0 μm. Details of the TOF-SIMS measurement method will be described later.

In the coated device produced, it is preferable that covalent bonding is not present between the hydrophilic polymer A and the device. The absence of covalent bonding enables the production of a coated device by a simpler process, regardless of whether the device is made of a water-containing material or a low-water-content material. Here, the absence of covalent bonding refers to a state where there is no chemically reactive group or no group resulting from a reaction of such a group between the hydrophilic polymer A and the device. The fact that there is no chemically reactive group or no group resulting from a reaction of such a group can be confirmed by an elemental analysis method such as electron energy loss spectroscopy, energy dispersive X-ray spectroscopy, and time-of-flight secondary ion mass spectrometry, or a composition analysis method. Examples of such a chemically reactive group include azetidinium groups, epoxy groups, isocyanate groups, aziridine groups, and azlactone groups.

It should be noted that in the resulting coated device, the hydrophilic polymer A may be present only on a part of the surface of the device, on the entire surface of either the front or back side, or on the entire surface of both sides.

In the coated device according to the present invention, it is preferable that at least part of the hydrophilic polymer A be present in a mixed state with the device in order to form a stronger coat. Here, the term "a mixed state of the hydrophilic

polymer A and the device" refers to a state in which elements derived from the hydrophilic polymer A and elements derived from the device are both detected in the same region. This can be determined by observing a cross-section of the coated device by an elemental analysis method such as scanning transmission electron microscopy, electron energy loss spectroscopy, energy dispersive X-ray spectroscopy, and time-of-flight secondary ion mass spectrometry, or a composition analysis method.

When the hydrophilic polymer A is dissolved in water, an appropriate degree of viscosity is developed, enabling the formation of a surface having not only hydrophilicity but also lubricity. Accordingly, the viscosity of the aqueous solution of the hydrophilic polymer A is preferably 1 to 2,000 mPa·s. The viscosity of the aqueous solution of the hydrophilic polymer A is more preferably 2 mPa·s or more, still more preferably 5 mPa·s or more, and particularly preferably 10 mPa·s or more. On the other hand, the viscosity is more preferably 1,900 mPa·s or less, still more preferably 1,000 mPa·s or less, and particularly preferably 500 mPa·s or less. The viscosity as referred to herein is a measured value of viscosity of a 2.0 to 3.5 mass% solution of the hydrophilic polymer A dissolved in water. The measuring method to use will be described in detail later.

To ensure enhanced adsorption of the hydrophilic polymer A to the surface of the device to impart sufficient lipid adhesion resistance to the device, it is preferable for the hydrophilic polymer A to have a weight average molecular weight of 2,000 to 1,500,000. Its weight average molecular weight is more preferably 5,000 or more, still more preferably 10,000 or more, and particularly preferably 100,000 or more. On the other hand, the weight average molecular weight is more preferably 1,000,000 or less, still more preferably 800,000 or less, and particularly preferably 600,000 or less. The weight average molecular weight as referred to herein is a weight average molecular weight converted in terms of polyethylene glycol, which is measured by gel permeation chromatography using an aqueous solvent as eluent. The measuring method to use will be described in detail later.

From the viewpoint of ensuring easy production of the coated device and high adsorptivity of the hydrophilic polymer A to the surface of the device, it is preferable that the hydrophilic polymer A have an appropriate filterability so that the hydrophilic polymer A can form a solution that can be filtered continuously. The measuring method to use will be described in detail later.

To ensure enhanced adsorption of the hydrophilic polymer A to the surface of the device to impart sufficient hydrophilicity and lubricity to the device, it is preferable that the change in the average particle diameter of the hydrophilic polymer A be irreversible when cooled again after heating. "Being irreversible" means that when a solution containing the hydrophilic polymer A is heated from 25°C to 121°C and then cooled back to 25°C, the average particle diameter of the hydrophilic polymer A increases by 10 nm or more compared to the state before heating, indicating its aggregation. It is more preferable that the increase in the average particle diameter of the hydrophilic polymer A that occurs when it is heated and then cooled back to 25°C is 40 nm or more, more preferably 70 nm or more, and particularly preferably 80 nm or more, because it allows the hydrophilic polymer A to form a stronger coat on the device. The measuring method to use will be described in detail later.

The electric charge of the hydrophilic polymer A is preferably within ±5 mV, which means that it has substantially no charge because the resulting coated device is very high in lipid and protein adhesion resistance. The measuring method to use will be described in detail later.

Furthermore, the hydrophilic polymer A that coats the surface of the device according to the present invention is preferably nonuniformly dispersed in a frozen state. "Being nonuniformly dispersed" means that regions stained by $RuO_4$ and regions unstained by $RuO_4$ are found to coexist when a frozen-state cross section of the hydrophilic polymer A that coats the surface of the device is observed by scanning transmission electron microscopy. The regions unstained by $RuO_4$ include regions and spaces filled with water and regions in which the hydrophilic polymer A has structures that cannot be stained by $RuO_4$. For example, in the case of using an ophthalmic lens as the device, lacrimal liquid components such as lipids and proteins tend to pass through more easily, and adhesion of these lacrimal liquid components is suppressed, leading to improved antifouling property of the coated device. Such regions in which the hydrophilic polymer A is nonuniformly dispersed may exist only in some parts of the hydrophilic polymer A or all parts of the hydrophilic polymer A. The measuring method to use will be described in detail later.

Meanwhile, the production method for the coated device according to the present invention includes a contact step (A) in which a device is placed in a container, followed by bringing the device into contact with a solution a of a hydrophilic polymer A and a heating step (C) in which the container is heated, wherein the hydrophilic polymer A contains, as monomer units, a compound a1 as represented by the general formula (I) given below, a compound a2 having an amide group, and a compound a3 having a plurality of hydroxyl groups, the polymerization ratio among the compound a1, compound a2, and compound a3 being such that the compound a1 accounts for 1 to 96 mass%, the compound a2 for 1 to 96 mass%, and the compound a3 for 8 to 61 mass%, and the solution a after the heating step having a pH in the range of 6.1 to 8.0.

[Chemical compound 6]

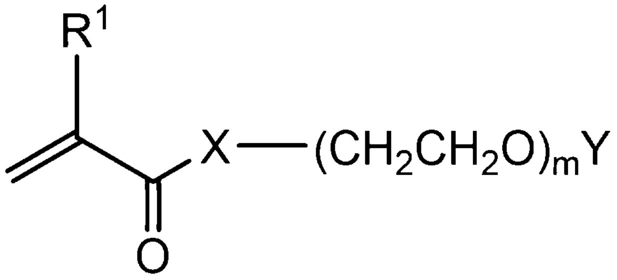

· · · (I)

[In the general formula (I), $R^1$ represents a hydrogen atom or a methyl group; X represents an oxygen atom or $NR^2$; $R^2$ represents a hydrogen atom or an alkyl group; m represents an integer of 1 to 30; and Y represents a hydrogen atom or an alkyl group.]

The content of the hydrophilic polymer A in the solution a is preferably 0.0001 to 30 mass% to allow the solution a to have a suitable viscosity. The content of the hydrophilic polymer A is preferably 0.001 mass% or more, more preferably 0.005 mass% or more, and still more preferably 0.05 mass% or more. On the other hand, the content of the hydrophilic polymer A is preferably 10.0 mass% or less, more preferably 5.0 mass% or less, still more preferably 1.0 mass% or less, and particularly preferably 0.5 mass% or less.

From the viewpoint of ease of handleability, the solvent to use for the solution a that contains the hydrophilic polymer A is preferably a water-soluble organic solvent, water, or a mixed solvent thereof. Of these, the use of a mixed solvent of a water-soluble organic solvent and water or the use of water is more preferable, and the use of water is still more preferable. The water-soluble organic solvent is preferably a water-soluble alcohol, more preferably a water-soluble alcohol having 6 or less carbon atoms, and still more preferably a water-soluble alcohol having 5 or less carbon atoms. The solution a may further contain a buffering agent or other additives.

Examples of the buffering agent contained in the solution a include boric acid, borates (for example, sodium borate), citric acid, citrates (for example, potassium citrate), bicarbonates (for example, sodium bicarbonate), phosphate buffers (for example, $Na_2HPO_4$, $NaH_2PO_4$, and $KH_2PO_4$), TRIS (tris(hydroxymethyl)aminomethane), 2-bis(2-hydroxyethyl)amino-2-(hydroxymethyl)-1,3-propanediol, bis-aminopolyols, triethanolamine, ACES (N-(2-acetamido)-2-aminoethanesulfonic acid), BES (N,N-bis(2-hydroxyethyl)-2-aminoethanesulfonic acid), HEPES (4-(2-hydroxyethyl)-1-piperazineethanesulfonic acid), MES (2-(N-morpholino)ethanesulfonic acid), MOPS (3-[N-morpholino]-propanesulfonic acid), PIPES (piperazine-N,N'-bis(2-ethanesulfonic acid)), and TES (N-[tris(hydroxymethyl)methyl]-2-aminoethanesulfonic acid), as well as salts thereof. The content of the buffering agent in the solution a is typically 0.001 to 2.000 mass%, though it may be adjusted appropriately to ensure a desired pH value. The content of the buffering agent is more preferably 0.010 mass% or more, still more preferably 0.050 mass% or more. On the other hand, the content of the buffering agent is more preferably 1.000 mass% or less, and still more preferably 0.300 mass% or less.

The pH of the buffer solution to use to prepare the solution a is preferably within the physiologically acceptable range of 6.3 to 7.8. The pH of the buffer solution is more preferably 6.5 or more, and still more preferably 6.8 or more. On the other hand, the pH of the buffer solution is more preferably 7.6 or less, and still more preferably 7.4 or less.

For example, in the case where the device is an ophthalmic lens, the container for storing the device in the contact step may be a vial or a blister container intended for packaging ophthalmic lenses. A blister container typically consists mainly of a plastic base part surrounded by a flat flange that stands around the edge of the cavity, and a soft cover sheet that is bonded to the flat flange to seal the cavity.

Examples of useful materials for the plastic base part include fluororesins, polyamides, polyacrylates, polyethylene, various nylons, various olefin copolymers (for example, copolymers of polypropylene and polyethylene), polyethylene terephthalate, polyvinyl chloride, amorphous polyolefins, polycarbonates, polysulfones, polybutylene terephthalate, polypropylenes, polymethylpentene, polyesters, various rubbers, and urethanes.

Examples of useful soft cover sheets include laminated materials such as polypropylene sheets coated with aluminum foil.

The production method for the coated device according to the present invention includes a heating step (C) for heating the aforementioned container.

Examples of useful heating methods include simple heating (hot air), high-pressure steam sterilization, dry heat sterilization, flame sterilization, boiling disinfection, streaming steam disinfection, electromagnetic irradiation (such as gamma rays and microwaves), ethylene oxide gas sterilization (EOG sterilization), and ultraviolet sterilization. The use of high-pressure steam sterilization is preferable due to its ability to impart sufficient lipid adhesion resistance to the device and its low production cost, and it is more preferable to adopt autoclave sterilization using an autoclave as apparatus.

The heating temperature is preferably 80°C to 200°C in order to impart a sufficient degree of lipid and protein adhesion resistance to the device while avoiding adverse effects on the strength of the resulting coated device. The heating temperature is more preferably 90°C or more, still more preferably 105°C or more, still more preferably 110°C or more, and particularly preferably 121°C or more. On the other hand, the heating temperature is more preferably 180°C or less, still more preferably 170°C or less, and particularly preferably 150°C or less.

For the same reason as for heating temperature, the heating time is preferably 1 to 600 minutes. The heating time is more preferably 2 minutes or more, still more preferably 5 minutes or more, and particularly preferably 10 minutes or more. On the other hand, the heating time is more preferably 400 minutes or less, still more preferably 300 minutes or less, and particularly preferably 100 minutes or less.

The production method for the coated device according to the present invention preferably includes a sealing step (B) for sealing the container that houses the device after the contact step and before the heating step. If the container that houses the device is sealed before the heating step, it serves not only to impart lipid and protein adhesion resistance to the surface of the device but also to sterilize the resulting coated device and maintain the sterilized state, which is industrially important for reducing the number of production steps. Specifically, for the production method for the coated device according to the present invention, the implementation of sterilization of the device through the aforementioned heating step is preferable for simplifying the production process. Examples of means for sealing the container here include the use of a capped vial or blister container as the container for housing the device, thereby sealing the container. When the device is a contact lens, furthermore, another means for sealing a container is to use a general-purpose lens case attached to contact lens care products as the container for housing the device.

After the above heating step, additional treatment may be performed on the resulting coated device. Examples of additional processing include similar simple heating using, for example, a buffer solution not containing a hydrophilic polymer, irradiation with rays such as ion beams, electron beams, positron beams, X-rays, gamma rays, and neutron rays, layer-by-layer (LbL) processing, which involves alternate coating with polymer materials having opposite charges (for example, the processing method described in International Publication No. WO2013/024800), and crosslinking with metal ions or chemical crosslinking (for example, the processing method described in Published Japanese Translation of PCT International Publication JP 2014-533381).

Furthermore, the surface of the device may be pretreated before the aforementioned contact and heating steps. Examples of such pretreatment include hydrolysis treatment with acids such as polyacrylic acid or alkalis such as sodium hydroxide (for example, the treatment method described in Japanese Patent No. 6954490).

The production method for the coated device according to the present invention requires that the pH of the solution a after the heating step be 6.1 to 8.0. If the pH is in this range, there is no need to wash the resulting coated device with a neutral solution after the heating step, which is industrially important from the perspective of reducing production steps. It should be noted that if washing with a neutral solution is performed after the heating step, it may become necessary to re-sterilize the resulting coated device. The pH of the solution a after the heating step is preferably 6.5 or more, more preferably 6.6 or more, still more preferably 6.7 or more, and particularly preferably 6.8 or more. On the other hand, the pH is preferably 7.9 or less, more preferably 7.8 or less, and still more preferably 7.6 or less.

It is also preferable that the pH of the solution a before the heating step is 6.1 to 8.0. Here, the pH of the solution a before the heating step refers to the value of pH measured after preparing a solution a and stirring the solution uniformly by operating a stirrer at room temperature (20°C to 23°C) for 30 minutes.

The pH of the solution a can be measured using a pH meter (for example, Eutech pH2700, manufactured by Eutech Instruments). Then, the measured value is rounded to the first decimal place.

In the case where the coated device is an ophthalmic lens or the like, it is preferable that a long liquid film retention time be ensured on the surface of the coated device, not only to prevent adhesion to the wearer's cornea but also to maintain good wearing sensation for a long time with reduced sensation of dryness.

Here, to determine the liquid film retention time on the surface of a coated device, the coated device is left to stand in a solution at room temperature (20°C to 23°C), then removed out of the solution and held in the air in such a manner that the longitudinal direction of the device aligns with the gravitational direction while measuring the time period during which the liquid film on the surface remains unbroken. In the case of a hemispherical device such as a contact lens, it is held so that the diameter direction of the circle formed by the edge of the spherical part aligns with the gravitational direction. "Breakage of the liquid film" means a state in which part of the solution covering the surface of the coated device is repelled so that the surface of the coated device becomes no longer covered completely by the liquid film. If the liquid film retention time is too long, it will allow the water evaporation from the surface of the coated device to progress too fast, resulting in deterioration in the hydrophilic effect. Accordingly, the liquid film retention time is preferably 300 seconds or less, and more preferably 200 seconds or less.

In the case where the coated device is an ophthalmic lens or the like, it is preferable that a small sessile droplet contact angle be ensured on the surface of the coated device, not only to prevent adhesion to the wearer's cornea but also to maintain good wearing sensation for a long time with reduced sensation of dryness. The method to use for measuring the sessile droplet contact angle will be described later. When the device contains silicon atoms, the sessile droplet contact

angle is preferably 80° or less, more preferably 70° or less, and still more preferably 65° or less.

In the case of a device free of silicon atoms, the sessile droplet contact angle is preferably 70° or less, more preferably 60° or less, and still more preferably 55° or less.

For example, when the coated device is one to be inserted in vivo, it is preferable that the surface of the coated device have high lubricity. Its coefficient of friction, which represents the degree of lubricity, is preferably 0.300 or less, more preferably 0.200 or less, still more preferably 0.100 or less, and still more preferably 0.080 or less. On the other hand, an excessively high lubricity can lead to deterioration in handleability of the coated device, and accordingly, its coefficient of friction is preferably 0.001 or more, and more preferably 0.002 or more. The method to use for measuring the coefficient of friction will be described later.

In the case where the coated device is a soft device such as an ophthalmic lens, the tensile elastic modulus is preferably 10.00 MPa or less, more preferably 5.00 MPa or less, still more preferably 3.00 MPa or less, still more preferably 2.00 MPa or less, still more preferably 1.00 MPa or less, and particularly preferably 0.60 MPa or less, in order to ensure good wearing sensation. On the other hand, in order to facilitate its handling, the tensile elastic modulus of the coated device is preferably 0.01 MPa or more, more preferably 0.10 MPa or more, still more preferably 0.20 MPa or more, and particularly preferably 0.25 MPa or more. The rate of change in the tensile elastic modulus between before and after the heating step, i.e., before and after coating, is preferably within ±15.00%, more preferably within ±13.00%, and still more preferably within ±10.00%, in order to reduce the risk of deformation or discomfort during wear. The method to use for measuring the tensile elastic modulus will be described later.

The antifouling property of the coated device according to the present invention can be evaluated based on the degree of mucin adhesion and lipid (methyl palmitate) adhesion. It is preferable that the amount of adhesion determined above be not larger than or less than its amount on the device itself, measured before coating, because this improves comfort during wear and reduces the risk of bacterial growth. The amount of mucin adhesion is preferably 10 $\mu g/cm^2$ or less, more preferably 8 $\mu g/cm^2$ or less, and particularly preferably 6 $\mu g/cm^2$ or less. The measuring method to use will be described later. In the case where the coated device is an ophthalmic lens, it is preferable that the change in water content of the device between before and after coating be 10 mass% or less, more preferably 8 mass% or less, and still more preferably 6 mass% or less, in order to prevent poor vision or deformation caused by distortion in refractive index due to increased water content. The method to use for measuring the water content will be described later.

In the case where the coated device is an ophthalmic lens, it is preferable that the change in size of the device between before and after coating be within ±5.00%, more preferably within ±4.00%, and still more preferably within ±3.00%, in order to prevent corneal damage due to its deformation.

## EXAMPLES

The present invention will be illustrated below in greater detail with reference to examples etc., although the invention should not be construed as being limited thereto. First, the analysis methods and evaluation methods used in the examples etc. are described below.

<Hydrophilicity (liquid film retention time)>

After the heating step, the coated device (or device) is allowed to stand until it reaches room temperature (20°C to 23°C), then removed from the solution in the container and held in the air with its longitudinal direction aligned with the gravitational direction. From the time period starting when the device is held in the air and ending when part of the liquid film covering the surface of the device is broken is visually observed. Three measurements (N = 3) were taken and their average was used for evaluations made according to the criteria specified below. The maximum measuring time was set to 120 seconds.

A: The liquid film on the surface is retained for 20 seconds or more.
B: The liquid film on the surface is broken in 15 seconds or more and less than 20 seconds.
C: The liquid film on the surface is broken in 10 seconds or more and less than 15 seconds.
C: The liquid film on the surface is broken in 1 second or more and less than 10 seconds.
D: The liquid film on the surface is broken in less than 1 second.

<Hydrophilicity after 26-hour immersion in fresh phosphate buffer solution (liquid film retention time measured after 26-hour immersion)>

To eliminate the influence of the hydrophilic polymer not firmly adsorbed on the surface of the device, the coated device or device after the heating step was immersed for 26 hours in 4 mL of a fresh phosphate buffer solution in a glass vial at room temperature (20°C to 23°C). The coated device or device was then removed from the phosphate buffer solution in a

glass vial. It was used as a sample (referred to as "sample S" below) and subjected to evaluations that were similar to those for hydrophilicity made above.

<Sessile droplet contact angle after 26-hour immersion in fresh phosphate buffer solution (sessile droplet contact angle X)>

Another sample S was prepared separately and subjected to measurement using a contact angle measurement device (droplet method) (Drop master DM500, manufactured by Kyowa Interface Science Co., Ltd.). More specifically, water was wiped off from the surface of the coated device (or device), and it was placed on a hemispherical polypropylene plate with a diameter of 14.5 mm to provide a sample. The sample was set on the contact angle measurement device, and a phosphate buffer solution was dropped to measure the contact angle. The droplet volume of the phosphate buffer solution was 20 μL. The contact angle was measured 30 seconds after the buffer dropping. Three measurements were taken (N = 3) and their average was adopted to represent the sessile droplet contact angle.

<Water content of device and coated device>

Prior to subjecting the device to the production method according to the present invention, it was immersed in a phosphate buffer solution and allowed to stand at room temperature (20°C to 23°C) for 24 hours or more. Subsequently, the device was removed from the phosphate buffer solution, and the surface water was wiped off using wiping cloth (Kimwipes (registered trademark), manufactured by Nippon Paper Crecia Co., Ltd.), followed by measuring the mass (Ww) of the device. Then, the device was dried at 40°C for 2 hours in a vacuum dryer, and the mass (Wd) of the device was measured. The water content of the device was calculated from these mass measurements by the equation (1) given below. Three measurements were taken (N = 3) and their average was adopted to represent the water content. The water content of the coated device obtained from the heating step was also calculated in the same manner.

$$\text{Water content of device (mass\%)} = 100 \times (Ww - Wd) / Ww \quad \ldots \text{ equation (1)}$$

<Change in water content of device between before and after coating>

Based on the measurements of the water content of the above device or coated device, the change in water content (percentage points) was calculated by the equation (2) given below.

Change in water content of device between before and after coating (percentage points) = water content of coated device (mass%) - water content of device (mass%) equation (2)

<Coefficient of friction>

The coefficient of friction of the surface of the coated device (or device) wetted with a phosphate buffer solution was measured from five samples (N = 5) under the conditions given below. The average of the five measurements (N = 5) was adopted to represent the coefficient of friction.

Equipment: frictional texture tester (KES-SE, manufactured by Kato Tech Co., Ltd.)

Friction SENS: H

$$\text{Measuring speed: } 2 \times 1 \text{ mm/s}$$

Friction load: 44 g

<Coefficient of friction after 26-hour immersion in fresh phosphate buffer solution (coefficient of friction X)>

Another sample S was prepared and evaluated in the same manner as for the coefficient of friction described above.

<Coefficient of friction after 1-minute rubbing following 26-hour immersion in fresh phosphate buffer solution (coefficient of friction Y)>

Another sample S was prepared and rubbed between fingers (between thumb and index finger) for 1 minute,

followed by making evaluations in the same manner as for the coefficient of friction described above.

<Detection depth of hydrophilic polymer>

For pretreatment, a phosphate buffer solution was dropped on the coated device (or device) from a pipette, followed by washing the surface lightly. Then, the coated device (or device) was fully immersed in 10 mL of a phosphate buffer solution in a glass vial and allowed to stand for 24 hours at room temperature (20°C to 23°C). Subsequently, it was air-dried on a clean Si wafer until droplets of the phosphate buffer solution were no longer visible, and then introduced into an ultra-high vacuum chamber.

The sample was subjected to measurement before and after the treatment under the conditions shown below.

Equipment: TOF.SIMS 5 (manufactured by ION-TOF)
Primary ion: $Bi_3^{++}$
Secondary ion polarity: positive only
Etchig ion: Ar-GCIB (gas cluster ion beam)

<Nonuniformity of hydrophilic polymer>

A frozen cross-section of the hydrophilic polymer that coated the surface of the device was observed under the following conditions to determine the nonuniformity of the hydrophilic polymer. If the hydrophilic polymer coating the device exhibited both RuOa-stained regions and $RuO_4$-unstained regions, it was judged to be nonuniform.

Sample preparation: conducted by the $RuO_4$-staining cryo-ultramicrotomy method. The coated device (or device) was immersed in a phosphate buffer solution for 24 hours to eliminate the influence of the hydrophilic polymer not sufficiently adsorbed to the surface of the device. Then, a frozen ultrathin section was prepared and introduced into the equipment using a cryo-transfer holder while maintaining the frozen state.

Observed site: central part of the coated device (or device)
Equipment: atomic-resolution analytical electron microscope (JEM-ARM200F Dual-X, manufactured by JEOL Ltd.)
Measuring conditions: accelerating voltage 200 kV

<Amount of lipid adhesion>

In a 20 cc screw tube, 0.03 g of methyl palmitate, 10 g of pure water, and the coated device (or device) were fed. The screw tube was shaken for 3 hours under the conditions of 37°C and 165 rpm. After shaking, the coated device (or device) in the screw tube was cleaned by rubbing with 40°C tap water and household liquid detergent (Mama Lemon (registered trademark), manufactured by Lion Corporation). The cleaned sample was put in a screw tube containing a phosphate buffer solution and stored in a refrigerator at 4°C for 24 hours. Then, the coated device (or device) was visually observed, and if any white turbid parts were found, they were considered to suffer from adhesion of methyl palmitate, and the area ratio of the methyl palmitate-adhered parts relative to the entire surface of the sample was determined.

<Amount of mucin adhesion>

Using a specified punching die, a test piece with a width (minimum breadth) of 5 mm and a length of 14 mm was cut out from the coated device (or device). Mucin, Bovine Submaxillary Gland (catalog number 499643, manufactured by CALBIOCHEM) was used as mucin for test. The test piece was immersed in an aqueous mucin solution with a concentration of 0.1% under the conditions of 20 hours and 37°C, and the amount of mucin adhesion to the sample was determined by the BCA (bicinchoninic acid) protein assay method. Three measurements were taken (N = 3) and their average was adopted to represent the amount of mucin adhesion.

<Tensile elastic modulus>

Using a specified punching die, a test piece with a width (minimum breadth) of 5 mm and a length of 14 mm was cut out from the coated device (or device). The test piece was subjected to a tensile test using a Tensilon RTG-1210 (manufactured by A&D Company, Limited). The tensile speed was 100 mm/min, and the (initial) distance between grips was 5 mm. Measurements were taken from both the device before contact and heating steps and the coated device after the contact and heating steps. Eight measurements were taken (N = 8) and, after excluding the maximum and the minimum, the average of the remaining six measurements (N = 6) was calculated to represent the tensile elastic modulus.

<Change in tensile elastic modulus of device between before and after coating>

From the tensile elastic modulus measurements taken above, the change in tensile elastic modulus of the device between before and after coating was calculated by the equation (3) given below.

Change in tensile elastic modulus of device between before and after coating (%) = (tensile elastic modulus of coated device after coating - tensile elastic modulus of device before coating)/tensile elastic modulus of device before coating × 100     equation (3)

<Size>

The length in the longitudinal direction (or the diameter in the case of a circular contact lens, etc.) of the coated device (or device) was measured for three test specimens (N = 3), and the average of the three measurements (N = 3) was calculated to represent the size.

<Change in size of device between before and after coating>

From the size measurements taken above, the change in size between before and after coating was calculated by the equation (4) given below.

Change in size between before and after coating (%) = (size of coated device after coating - size of device before coating) / size of device before coating × 100     equation (4)

<Measurement of weight average molecular weight>

The weight average molecular weight (hereinafter referred to as Mw) of the hydrophilic polymer was measured under the conditions given below.

Equipment: Prominence GPC System (manufactured by Shimadzu Corporation)
Pump: LC-20AD
Autosampler: SIL-20AV HT
Column oven: CTO-20A
Detector: RID-10A
Column: GMPWXL (manufactured by Tosoh Corporation, inner diameter 7.8 mm × 30 cm, particle diameter 13 μm)
Solvent: water/methanol = 1/1 (with 0.1 N lithium nitrate added)
Flow rate: 0.5 mL/min
Measuring time: 60 minutes
Sample concentration: 0.1 mass%
Sample filtration: filtered through a syringe filter with a pore size of 0.8 μm (DISMIC 25CS080AN, manufactured by ADVANTEC Toyo Co., Ltd.) and a syringe filter with a pore size of 0.45 μm (DISMIC 13HP045AN, manufactured by ADVANTEC Toyo Co., Ltd.)
Injected sample volume: 10 to 20 μL
Standard sample: polyethylene oxide standard sample (manufactured by Agilent, 0.1 to 1,258 kD)

<Measurement of viscosity>

Using an SV-10H viscometer (manufactured by A&D Co., Ltd.), the viscosities of 2 to 3.5 mass% aqueous solutions of the hydrophilic polymer were measured.

<pH>

The pH values of the solutions were measured using a pH meter (Eutech pH2700, manufactured by Eutech Instruments). The "post-heating pH" of the solution a was measured immediately after cooling it to room temperature (20°C to 23°C) following the heating step.

<Phosphate buffer solution>

The components of the phosphate buffer solution used in Examples and Comparative examples were as

specified below.

KCl: 0.2 g/L
$KH_2PO_4$: 0.2 g/L
NaCl: 8.0 g/L
$Na_2HPO_4$: 1.19 g/L
$EDTA_2Na$ (disodium dihydrogen ethylenediaminetetraacetate dihydrate): 0.5 g/L

<Filterability of hydrophilic polymer>

The hydrophilic polymer was dissolved in the phosphate buffer solution to a concentration of 0.1 mass%. The filterability of 50 g of this hydrophilic polymer solution was evaluated based on the degree of clogging that occurred during suction filtration measured using a 0.45 μm bottle-top filter (model 295-4545, manufactured by Thermo Fisher Scientific Inc.). If the solution passed through the filter continuously, it was judged to be good, whereas if only less than one drop per 5 seconds was allowed to pass, it was judged to be poor.

<Polymerization ratio of hydrophilic polymer>

To calculate the polymerization ratio of the hydrophilic polymer, the aqueous solution of the hydrophilic polymer was freeze-dried and subjected to acetonitrile extraction to remove unreacted monomers, followed by performing $^1$HNMR analysis. The polymerization ratio of the constituents was calculated from the peak area ratio in the $^1$HNMR spectrum.

<Hydrophilic polymer concentration of aqueous hydrophilic polymer solution>

To determine the hydrophilic polymer concentration of the aqueous hydrophilic polymer solution after polymerization, the aqueous hydrophilic polymer solution was freeze-dried and subjected to acetonitrile extraction to remove unreacted monomers, and the mass of the resulting acetonitrile-insoluble component was measured, followed by making a calculation by the equation (5) given below.

Hydrophilic polymer concentration (mass%) = mass of acetonitrile-insoluble component/ mass of sampled aqueous polymer solution × 100 equation (5)

<Average particle diameter of hydrophilic polymer>

A 0.2 mass% solution was prepared by diluting the hydrophilic polymer with a phosphate buffer solution and filtering it through a 0.45 μm filter. The resulting liquid was subjected to measurement under the conditions given below. Particle diameter distribution was measured at various temperatures by the dynamic light scattering method.

Name of equipment: Dynapro NanoStar (manufactured by Wyatt Technology)
Light source: semiconductor laser (wavelength 658 nm)
Measuring temperature: 25°C, re-measured after cooling from 121 °C* to 25°C
Analysis method: CONTIN method
Number of measurements (n): 2 (another solution was prepared for the second measuring run)
*: Measurement was performed at 121 °C by the following procedure to prevent evaporation of the solvent.

(1) Confirm that the setting part of the measuring cell has reached the desired measuring temperature.
(2) Set the measuring cell in the equipment and wait for 10 minutes before starting measurement.
(3) After measurement, remove the cell from the equipment.

<Electric charge of hydrophilic polymer>

The zeta potential of the hydrophilic polymer solution in a neutral state was measured by laser Doppler electrophoresis under the conditions given below.

Equipment: ELSZneo electrophoretic light scattering photometer (manufactured by Otsuka Electronics Co., Ltd.)
Light source: semiconductor laser (wavelength 660 nm)
Cell: standard flow cell
Voltage: 60 V

Measuring temperature: 25°C
Dispersion medium: 1 mM aqueous NaCl solution
Number of measurements: 2 (Two measuring solutions were prepared and 5 measurements were taken for each measuring solution.) Average of 10 measurements is adopted to represent the charge.
Judgment: If it is within ±5 mV, the sample is judged to have no charge.

(Synthesis example 1)

In a 500 mL four-necked flask equipped with a stirrer, thermometer, condenser tube, and three-way stopcock, 58.15 g of distilled water, 1.13 g of polyalkylene glycol monomethacrylate with side chain terminal methyl (PME-100, manufactured by NOF Corporation, polyethylene glycol chain number = 2 (m = 2 in equation (I), manufacturer-disclosed average), 6 mmol), 2.58 g of N,N-dimethylacrylamide (manufactured by FUJIFILM Wako Pure Chemical Corporation, 26 mmol), and 1.28 g of glycerol methacrylate (GLM, manufactured by NOF Corporation, 8 mmol) were fed, and ultrasonic degassing and nitrogen substitution were performed five times repeatedly. After completing nitrogen substitution in the system, the bottom part of the flask was immersed in an oil bath set at 85°C while stirring. When the internal temperature exceeded 70°C, 2.7 mg of a polymerization initiator (V-50, manufactured by FUJIFILM Wako Pure Chemical Corporation, 0.01 mmol) dissolved in 20.00 g of distilled water was added in five equal portions at 5-minute intervals. After the polymerization initiator was added completely, the internal temperature in the flask was maintained at 70°C to 75°C for 4 hours to obtain an aqueous solution of a copolymer of polyalkylene glycol methacrylate with side chain terminal methyl (PME-100) / N,N-dimethylacrylamide / glycerol methacrylate (polymerization ratio by mole 15/65/20, weight average molecular weight 200,000, viscosity of a 2.3 mass% aqueous solution 2.7 mPa·s).

(Synthesis example 2)

In a 500 mL four-necked flask equipped with a stirrer, thermometer, condenser tube, and three-way stopcock, 38.13 g of distilled water, 0.75 g of polyalkylene glycol monomethacrylate with side chain terminal methyl (PME-100, 4 mmol), 2.38 g of N,N-dimethylacrylamide (manufactured by FUJIFILM Wako Pure Chemical Corporation, 24 mmol), and 1.92 g of glycerol methacrylate (GLM, 12 mmol) were fed, and ultrasonic degassing and nitrogen substitution were performed five times repeatedly. After completing nitrogen substitution in the system, the bottom part of the flask was immersed in an oil bath set at 85°C while stirring. When the internal temperature exceeded 70°C, 2.7 mg of a polymerization initiator (V-50) dissolved in 20.00 g of distilled water was added in five equal portions at 5-minute intervals. After the polymerization initiator was added completely, the internal temperature in the flask was maintained at 70°C to 75°C for 4 hours to obtain an aqueous solution of a copolymer of polyalkylene glycol methacrylate with side chain terminal methyl / N,N-dimethylacrylamide / glycerol methacrylate (polymerization ratio by mole 10/60/30, weight average molecular weight 210,000, viscosity of a 2.3 mass% aqueous solution 1.7 mPa·s).

(Synthesis example 3)

In a 100 mL three-neck flask, 3.20 g of methoxytetraethylene glycol methacrylate (BRENMER (registered trademark) PME-200, manufactured by NOF Corporation, 11.6 mmol), 50.1 mg of 2,2'-azobis[2-(2-imidazolin-2-yl) propane] as a polymerization initiator (VA-061, manufactured by Wako Pure Chemical Industries, Ltd., 0.200 mmol), and 28.23 g of distilled water (manufactured by FUJIFILM Wako Pure Chemical Corporation) were fed, and then a digital thermometer, a Dimroth condenser tube with a three-way stopcock, and a sealer with stirring blades were attached. While applying ultrasonic waves, a cycle of evacuation down to 10 mmHg and nitrogen flushing was repeated five times to remove dissolved oxygen from the mixed solution. Subsequently, the liquid was stirred and reacted in an oil bath at 60°C for 7 hours, and then the reaction vessel was removed from the oil bath and air-cooled. After reducing the viscosity by adding 50 mL of methanol to the polymerization reaction solution and stirring, the liquid was transferred to a 1 L Teflon (registered trademark) beaker and heated for drying overnight at 40°C in a vacuum dryer. After drying, 5 mL of isopropanol (IPA) was added to dissolve the resulting viscous solid material, and the resulting solution was allowed to stand in a refrigerator overnight. After cooling, the supernatant was removed by decantation, and drying was performed at 30°C for 2 hours in a vacuum dryer to obtain a white powdery polymer. The weight average molecular weight of the resulting methoxypolyethylene glycol methacrylate homopolymer (ethylene oxide repeating unit number = 4) was 250,000.

(Synthesis example 4)

In a 500 mL four-necked flask equipped with a stirrer, thermometer, condenser tube, and three-way stopcock, 82.18 g of distilled water, 3.97 g of polyalkylene glycol monomethacrylate with side chain terminal methyl (PME-400, manufactured by NOF Corporation, polyethylene glycol chain number = 9 (m = 9 in equation (I), manufacturer-disclosed

average), 8 mmol), 6.34 g of N,N-dimethylacrylamide (manufactured by FUJIFILM Wako Pure Chemical Corporation, 64 mmol), and 1.04 g of 2-hydroxyethyl methacrylate (manufactured by FUJIFILM Wako Pure Chemical Corporation, 8 mmol) were fed, and ultrasonic degassing and nitrogen substitution were performed five times repeatedly. After completing nitrogen substitution in the system, the bottom part of the flask was immersed in an oil bath set at 85°C while stirring. When the internal temperature exceeded 70°C, 5.4 mg of a polymerization initiator (V-50, 0.02 mmol) dissolved in 20.00 g of distilled water was added in five equal portions at 5-minute intervals. After the polymerization initiator was added completely, the internal temperature in the flask was maintained at 80°C to 85°C for 4 hours to obtain an aqueous solution of a copolymer of polyalkylene glycol methacrylate with side chain terminal methyl / N,N-dimethylacrylamide / 2-hydroxyethyl methacrylate (polymerization ratio by mole 10/80/10, weight average molecular weight 300,000, viscosity of a 3.4 mass% aqueous solution 50 mPa·s).

(Synthesis example 5)

In a 500 mL four-necked flask equipped with a stirrer, thermometer, condenser tube, and three-way stopcock, 34.52 g of tert-amyl alcohol (manufactured by FUJIFILM Wako Pure Chemical Corporation), 9.22 g of polyalkylene glycol monomethacrylate with side chain terminal methyl (PME-100, 49 mmol), 4.96 g of N,N-dimethylacrylamide (manufactured by FUJIFILM Wako Pure Chemical Corporation, 50 mmol), 0.61 g of silicone monomer $T_1$ as represented by the chemical formula (II) given below (manufactured by Toray Industries, Inc., 1 mmol), and 0.0248 g of polymerization initiator 2,2-azobis(2,4-dimethylvaleronitrile) (manufactured by FUJIFILM Wako Pure Chemical Corporation, 0.1 mmol) were fed, and ultrasonic degassing and nitrogen substitution were performed five times repeatedly. After completing nitrogen substitution in the system, the bottom part of the flask was immersed in an oil bath set at 85°C while stirring. The internal temperature in the flask was maintained at 70°C to 75°C for 4 hours to obtain an aqueous solution of a copolymer of polyalkylene glycol monomethacrylate with side chain terminal methyl / N,N-dimethylacrylamide / silicone monomer $T_1$. After reducing the viscosity by adding 43 mL of ethanol to the resulting aqueous copolymer solution and stirring, the solution was dropped into 740 mL of hexane to precipitate the copolymer.

The precipitated copolymer was heated and dried in a vacuum dryer at 60°C for 4 hours. The polymerization ratio by mole of the resulting copolymer was 49/50/1. Due to incomplete dissolution, the copolymer failed to be dissolved completely in the measuring solvent, and therefore, it was impossible to measure its weight average molecular weight and viscosity.

[Chemical compound 7]

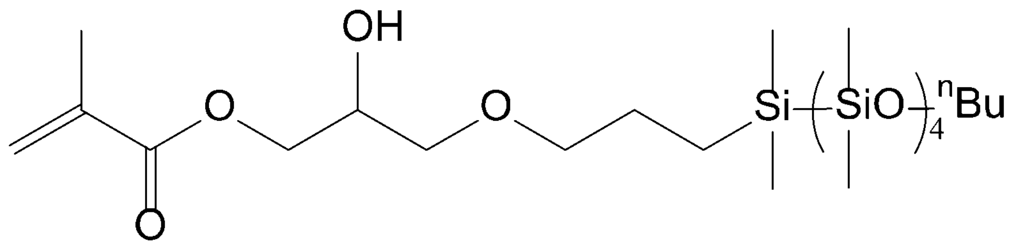

・・・(II)

(Synthesis example 6)

In a 500 mL four-necked flask equipped with a stirrer, thermometer, condenser tube, and three-way stopcock, 34.07 g of tert-amyl alcohol (manufactured by FUJIFILM Wako Pure Chemical Corporation), 9.22 g of polyalkylene glycol monomethacrylate with side chain terminal methyl (PME-100, 49 mmol), 4.96 g of N,N-dimethylacrylamide (manufactured by FUJIFILM Wako Pure Chemical Corporation, 50 mmol), 0.42 g of silicone monomer $T_2$ as represented by the chemical formula (III) given below (manufactured by Toray Industries, Inc., 1 mmol), and 0.0248 g of polymerization initiator 2,2-azobis(2,4-dimethylvaleronitrile) (manufactured by FUJIFILM Wako Pure Chemical Corporation, 0.1 mmol) were fed, and ultrasonic degassing and nitrogen substitution were performed five times repeatedly. After completing nitrogen substitution in the system, the bottom part of the flask was immersed in an oil bath set at 85°C while stirring. The internal temperature in the flask was maintained at 70°C to 75°C for 4 hours to obtain an aqueous solution of a copolymer of polyalkylene glycol monomethacrylate with side chain terminal methyl / N,N-dimethylacrylamide / silicone monomer $T_2$. After reducing the viscosity by adding 43 mL of ethanol to the resulting aqueous copolymer solution and stirring, the solution was dropped into 740 mL of hexane to precipitate the copolymer. The precipitated polymer was heated and dried in a vacuum dryer at 60°C for 4 hours. The polymerization ratio by mole of the resulting copolymer was 49/50/1. Due to incomplete dissolution, the copolymer failed to be dissolved completely in the measuring solvent, and therefore, it was

impossible to measure its weight average molecular weight and viscosity.

[Chemical compound 8]

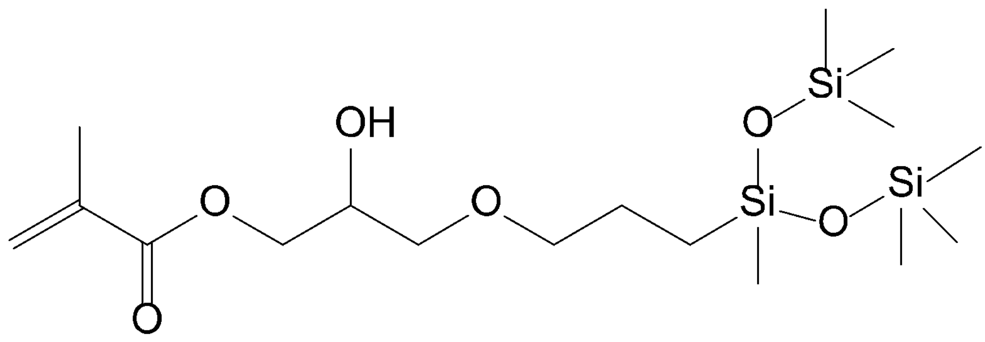

・・・(III)

(Synthesis example 7)

In a 500 mL four-necked flask equipped with a stirrer, thermometer, condenser tube, and three-way stopcock, 45.15 g of distilled water, 0.75 g of polyalkylene glycol monomethacrylate with side chain terminal methyl (PME-100, 4 mmol), 1.39 g of N,N-dimethylacrylamide (manufactured by FUJIFILM Wako Pure Chemical Corporation, 14 mmol), and 3.52 g of glycerol methacrylate (GLM, 22 mmol) were fed, and ultrasonic degassing and nitrogen substitution were performed five times repeatedly. After completing nitrogen substitution in the system, the bottom part of the flask was immersed in an oil bath set at 85°C while stirring. When the internal temperature exceeded 70°C, 2.7 mg of a polymerization initiator (V-50, 0.01 mmol) dissolved in 20.00 g of distilled water was added in five equal portions at 5-minute intervals. After the polymerization initiator was added completely, the internal temperature in the flask was maintained at 70°C to 75°C for 4 hours to obtain an aqueous solution of a copolymer of polyalkylene glycol methacrylate with side chain terminal methyl / N,N-dimethylacrylamide / glycerol methacrylate (polymerization ratio by mole 10/35/55, weight average molecular weight 220,000, viscosity of a 3.4 mass% aqueous solution 50 mPa·s).

(Synthesis example 8)

In a 500 mL four-necked flask equipped with a stirrer, thermometer, condenser tube, and three-way stopcock, 33.15 g of distilled water, 1.13 g of polyalkylene glycol monomethacrylate with side chain terminal methyl (PME-100, 6 mmol), 3.17 g of N,N-dimethylacrylamide (manufactured by FUJIFILM Wako Pure Chemical Corporation, 32 mmol), and 0.32 g of glycerol methacrylate (GLM, 2 mmol) were fed, and ultrasonic degassing and nitrogen substitution were performed five times repeatedly. After completing nitrogen substitution in the system, the bottom part of the flask was immersed in an oil bath set at 85°C while stirring. When the internal temperature exceeded 70°C, 2.7 mg of a polymerization initiator (V-50, 0.01 mmol) dissolved in 20.00 g of distilled water was added in five equal portions at 5-minute intervals. After the polymerization initiator was added completely, the internal temperature in the flask was maintained at 70°C to 75°C for 4 hours to obtain an aqueous solution of a copolymer of polyalkylene glycol methacrylate with side chain terminal methyl / N,N-dimethylacrylamide / glycerol methacrylate (polymerization ratio by mole 15/80/5, weight average molecular weight 230,000, viscosity of a 2.3 mass% aqueous solution 15 mPa·s).

(Synthesis example 9)

In a 500 mL four-necked flask equipped with a stirrer, thermometer, condenser tube, and three-way stopcock, 46.17 g of distilled water, 2.07 g of polyalkylene glycol monomethacrylate with side chain terminal methyl (PME-200, manufactured by NOF Corporation, polyethylene glycol chain number = 4 (m = 4 in equation (I), manufacturer-disclosed average), 7.5 mmol), 2.48 g of N,N-dimethylacrylamide (manufactured by FUJIFILM Wako Pure Chemical Corporation, 25 mmol), and 2.80 g of glycerol methacrylate (GLM-EX, manufactured by NOF Corporation, 17.5 mmol) were fed, and ultrasonic degassing and nitrogen substitution were performed five times repeatedly. After completing nitrogen substitution in the system, the bottom part of the flask was immersed in an oil bath set at 85°C while stirring. When the internal temperature exceeded 70°C, 3.4 mg of a polymerization initiator (V-50, manufactured by FUJIFILM Wako Pure Chemical Corporation, 0.0125 mmol) dissolved in 20.00 g of distilled water was added in five equal portions at 5-minute intervals. After the polymerization initiator was added completely, the internal temperature in the flask was maintained at 70°C to 75°C for 4 hours to obtain an aqueous solution of a copolymer of polyalkylene glycol monomethacrylate with side chain

terminal methyl (PME-200) / N,N-dimethylacrylamide / glycerol methacrylate (polymerization ratio by mole 15/50/35, weight average molecular weight 290,000, viscosity of a 3.5 mass% aqueous solution 1,040 mPa·s).

(Synthesis example 10)

In a 500 mL four-necked flask equipped with a stirrer, thermometer, condenser tube, and three-way stopcock, 44.34 g of distilled water, 1.13 g of polyalkylene glycol monomethacrylate with side chain terminal methyl (PME-100, manufactured by NOF Corporation, polyethylene glycol chain number = 2 (m = 2 in equation (I), manufacturer-disclosed average), 6 mmol), 2.22 g of N-vinylpyrrolidone (manufactured by FUJIFILM Wako Pure Chemical Corporation, 20 mmol), and 2.24 g of glycerol methacrylate (GLM-EX, manufactured by NOF Corporation, 14 mmol) were fed, and ultrasonic degassing and nitrogen substitution were performed five times repeatedly. After completing nitrogen substitution in the system, the bottom part of the flask was immersed in an oil bath set at 85°C while stirring. When the internal temperature exceeded 70°C, 2.7 mg of a polymerization initiator (V-50, manufactured by FUJIFILM Wako Pure Chemical Corporation, 0.01 mmol) dissolved in 20.00 g of distilled water was added in five equal portions at 5-minute intervals. After the polymerization initiator was added completely, the internal temperature in the flask was maintained at 70°C to 75°C for 4 hours to obtain an aqueous solution of a copolymer of polyalkylene glycol monomethacrylate with side chain terminal methyl (PME-100) / N-vinylpyrrolidone / glycerol methacrylate (polymerization ratio by mole 15/50/35, weight average molecular weight 300,000, viscosity of a 2.3 mass% aqueous solution 10.6 mPa·s).

(Synthesis example 11)

In a 500 mL four-necked flask equipped with a stirrer, thermometer, condenser tube, and three-way stopcock, 32.14 g of distilled water, 0.85 g of polyalkylene glycol monomethacrylate with side chain terminal methyl (PME-100, manufactured by NOF Corporation, polyethylene glycol chain number = 2 (m = 2 in equation (I), manufacturer-disclosed average), 4.5 mmol), 1.64 g of N,N-dimethylacrylamide (manufactured by FUJIFILM Wako Pure Chemical Corporation, 16.5 mmol), and 1.44 g of glycerol methacrylate (GLM, manufacturered by NOF Corporation, 9 mmol) were fed, and ultrasonic degassing and nitrogen substitution were performed five times repeatedly. After completing nitrogen substitution in the system, the bottom part of the flask was immersed in an oil bath set at 85°C while stirring. When the internal temperature exceeded 70°C, 2.0 mg of a polymerization initiator (V-50, manufactured by FUJIFILM Wako Pure Chemical Corporation, 0.0075 mmol) dissolved in 20.00 g of distilled water was added in five equal portions at 5-minute intervals. After the polymerization initiator was added completely, the internal temperature in the flask was maintained at 70°C to 75°C for 4 hours to obtain an aqueous solution of a copolymer of polyalkylene glycol methacrylate with side chain terminal methyl (PME-100) / N,N-dimethylacrylamide / glycerol methacrylate (polymerization ratio by mole 15/55/30, weight average molecular weight 370,000, viscosity of a 2.3 mass% aqueous solution 10.4 mPa·s).

[Example 1]

A commercially available silicone hydrogel lens containing silicone as the main component, namely, "Ultra One Day" (registered trademark) (manufactured by Bausch & Lomb, kalifilcon A), was used as the "device"; a glass vial was used as the "container"; and a 3 ml of a 0.2 mass% solution of the hydrophilic polymer A prepared in Synthesis example 1, namely, a PME-100 / N,N-dimethylacrylamide / glycerol methacrylate copolymer, dissolved in the phosphate buffer solution, was used as the "solution a". The device was immersed in the solution a (contact step (A)), and the glass vial was sealed with a cap (sealing step (B)) and then heated in an autoclave at 121°C for 10 minutes (heating step (C)). The resulting coated device was evaluated according to the method described above, and results obtained are shown in Tables 1 to 3.

[Example 2]

Except that the device used was a commercially available silicone hydrogel lens containing polyvinylpyrrolidone and silicone as the main components, namely, "Acuvue Oasys" (registered trademark) (manufactured by Johnson & Johnson, senofilcon A), the same procedure as in Example 1 was carried out. The resulting coated device was evaluated according to the method described above, and results obtained are shown in Tables 1 to 3.

[Example 3]

Except that the device used was a commercially available hydrogel lens containing 2-hydroxyethyl methacrylate as the main component, namely, "Medalist One Day Plus" (registered trademark) (manufactured by Bausch & Lomb, hilafilcon B), the same procedure as in Example 1 was carried out. The resulting coated device was evaluated according to

the method described above, and results obtained are shown in Tables 1 to 3.

[Example 4]

Except that a 0.3 mass% solution of the hydrophilic polymer A prepared in Synthesis example 2, namely, a PME-100 / N,N-dimethylacrylamide / glycerol methacrylate copolymer, dissolved in the phosphate buffer solution, was used instead of the solution a, and that the heating in an autoclave was performed at 121 °C for 30 minutes, the same procedure as in Example 1 was carried out. The resulting coated device was evaluated according to the method described above, and results obtained are shown in Tables 1 to 3.

[Example 5]

Except that the device used was a commercially available silicone hydrogel lens, namely, "Acuvue Oasys" (registered trademark), the same procedure as in Example 4 was carried out. The resulting coated device was evaluated according to the method described above, and results obtained are shown in Tables 1 to 3.

[Example 6]

Except that the device used was a commercially available hydrogel lens, namely, "Medalist One Day Plus" (registered trademark), the same procedure as in Example 4 was carried out. The resulting coated device was evaluated according to the method described above, and results obtained are shown in Tables 1 to 3.

[Example 7]

Except that the device used was a commercially available silicone hydrogel lens containing polyvinylpyrrolidone and silicone as the main components, namely, "1day Acuvue TruEye" (registered trademark) (manufactured by Johnson & Johnson, narafilcon A), that a 0.2 mass% solution of the hydrophilic polymer A prepared in Synthesis example 9, namely, a PME-200 / N,N-dimethylacrylamide / glycerol methacrylate copolymer, dissolved in the phosphate buffer solution, was used instead of the solution a, and that the heating in an autoclave was performed at 121°C for 30 minutes, the same procedure as in Example 1 was carried out. The resulting coated device was evaluated according to the method described above, and results obtained are shown in Tables 1 to 3.

[Example 8]

Except that the device used was a commercially available silicone hydrogel lens, namely, "Acuvue Oasys" (registered trademark), the same procedure as in Example 7 was carried out. The resulting coated device was evaluated according to the method described above, and results obtained are shown in Tables 1 to 3.

[Example 9]

Except that a 0.3 mass% solution of the hydrophilic polymer A prepared in Synthesis example 10, namely, a PME-100 / N-vinylpyrrolidone / glycerol methacrylate copolymer, dissolved in the phosphate buffer solution, was used instead of the solution a, the same procedure as in Example 7 was carried out. The resulting coated device was evaluated according to the method described above, and results obtained are shown in Tables 1 to 3.

[Example 10]

Except that the device used was a commercially available silicone hydrogel lens, namely, "Acuvue Oasys" (registered trademark), the same procedure as in Example 9 was carried out. The resulting coated device was evaluated according to the method described above, and results obtained are shown in Tables 1 to 3.

[Example 11]

Except that the device used was a commercially available silicone hydrogel lens, namely, "1day Acuvue TruEye" (registered trademark) and that the hydrophilic polymer A prepared in Synthesis example 11, namely, a PME-100 / N,N-dimethylacrylamide / glycerol methacrylate copolymer was used instead of the solution a, the same procedure as in Example 1 was carried out. The resulting coated device was evaluated according to the method described above, and results obtained are shown in Tables 1 to 3.

[Example 12]

Except that the device used was a commercially available silicone hydrogel lens containing polyvinylpyrrolidone and silicone as the main components, namely, "1day Acuvue Oasys" (registered trademark) (manufactured by Johnson & Johnson, senofilcon A), that a 0.07 mass% solution of a hydrophilic polymer A manufactured by Osaka Organic Chemical Industry Ltd., namely, an aqueous solution of a copolymer of PME-100 / N,N-dimethylacrylamide / glycerol methacrylate (polymerization ratio by mole 24/27/49, weight average molecular weight 180,000, viscosity of a 2.3 mass% aqueous solution 10.7 mPa·s), dissolved in the phosphate buffer solution, was used instead of the solution a, and that the heating in an autoclave was performed at 121°C for 14 minutes, the same procedure as in Example 1 was carried out. The resulting coated device was evaluated according to the method described above, and results obtained are shown in Tables 1 to 3.

[Example 13]

Except that the device used was a commercially available silicone hydrogel lens, namely, "1day Acuvue TruEye" (registered trademark), the same procedure as in Example 12 was carried out. The resulting coated device was evaluated according to the method described above, and results obtained are shown in Tables 1 to 3.

[Example 14]

Except that a solution of a hydrophilic polymer A manufactured by Osaka Organic Chemical Industry Ltd., namely, an aqueous solution of a copolymer of PME-100 / N,N-dimethylacrylamide / glycerol methacrylate (polymerization ratio by mole 24/27/49, weight average molecular weight 510,000, viscosity of a 2.3 mass% aqueous solution 10.9 mPa·s) was used instead of the solution a, the same procedure as in Example 12 was carried out. The resulting coated device was evaluated according to the method described above, and results obtained are shown in Tables 1 to 3.

[Example 15]

Except that the device used was a commercially available silicone hydrogel lens, namely, "1day Acuvue TruEye" (registered trademark), the same procedure as in Example 14 was carried out. The resulting coated device was evaluated according to the method described above, and results obtained are shown in Tables 1 to 3.

[Table 1-1]

[Table 1-1]

| | device | water content of device (mass%) | solution of hydrophilic polymer | hydrophilic polymer polymerization ratio compound a1/compound a2/compound a3 |
|---|---|---|---|---|
| Example 1 | silicone hydrogel lens | 55.0 | 0.20 mass% polyalkylene glycol monomethacrylate with side chain terminal methyl /N,N-dimethylacrylamide/glycerol methacrylate copolymer | 22/52/26 |
| Example 2 | silicone hydrogel lens | 38.0 | 0.20 mass% polyalkylene glycol monomethacrylate with side chain terminal methyl /N,N-dimethylacrylamide/plycerol methacrylate copolymer | 22/52/26 |
| Example 3 | hydrogel lens | 59.0 | 0.20 mass% polyalkylene glycol monomethacrylate with side chain terminal methyl /N,N-dimethylacrylamide/plycerol methacrylate copolymer | 22/52/26 |
| Example 4 | silicone hydrogel lens | 55.0 | 0.30 mass% polyalkylene glycol monomethacrylate with side chain terminal methyl /N,N-dimethylacrylamide/glycerol methacrylate copolymer | 15/47/38 |

(continued)

| | device | water content of device (mass%) | solution of hydrophilic polymer | hydrophilic polymer polymerization ratio compound a1/compound a2/compound a3 |
|---|---|---|---|---|
| Example 5 | silicone hydrogel lens | 38.0 | 0.30 mass% polyalkylene glycol monomethacrylate with side chain terminal methyl /N,N-dimethylacrylamide/glycerol methacrylate copolymer | 15/47/38 |
| Example 6 | hydrogel lens | 59.0 | 0.30 mass% polyalkylene glycol monomethacrylate with side chain terminal methyl /N,N-dimethylacrylamide/plycerol methacrylate copolymer | 15/47/38 |
| Example 7 | silicone hydrogel lens | 46.0 | 0.20 mass% polyalkylene glycol monomethacrylate with side chain terminal methyl /N,N-dimethylacrylamide/glycerol methacrylate copolymer | 28/34/38 |
| Example 8 | silicone hydrogel lens | 38.0 | 0.20 mass% polyalkylene glycol monomethacrylate with side chain terminal methyl /N,N-dimethylacrylamide/glycerol methacrylate copolymer | 28/34/38 |
| Example 9 | silicone hydrogel lens | 46.0 | 0.30 mass% polyalkylene glycol monomethacrylate with side chain terminal methyl /N-vinylpyrrolidone/glycerol methacrylate copolymer | 20/40/40 |
| Example 10 | silicone hydrogel lens | 38.0 | 0.30 mass% polyalkylene glycol monomethacrylate with side chain terminal methyl /N-vinylpyrrolidone/glycerol methacrylate copolymer | 20/40/40 |
| Example 11 | silicone hydrogel lens | 46.0 | 0.20 mass% polyalkylene glycol monomethacrylate with side chain terminal methyl /N,N-dimethvlacrylamide/plycerol methacrvlate copolymer | 22/42/36 |
| Example 12 | silicone hydrogel lens | 38.0 | 0.07 mass% polyalkylene glycol monomethacrylate with side chain terminal methyl /N,N-dimethylacrylamide/glycerol methacrylate copolymer | 30/18/52 |
| Example 13 | silicone hydrogel lens | 46.0 | 0.07 mass% polyalkylene glycol monomethacrylate with side chain terminal methyl /N,N-dimethylacrylamide/glycerol methacrylate copolymer | 30/18/52 |
| Example 14 | silicone hydrogel lens | 38.0 | 0.07 mass% polyalkylene glycol monomethacrylate with side chain terminal methyl /N,N-dimethylacrylamide/glycerol methacrvlate copolymer | 30/18/52 |
| Example 15 | silicone hydrogel lens | 46.0 | 0.07 mass% polyalkylene glycol monomethacrylate with side chain terminal methyl /N,N-dimethylacrylamide/plycerol methacrylate copolymer | 30/18/52 |

[Table 1-2]

[Table 1-2]

| | filterability of hydrophilic polymer | average particle diameter of hydrophilic polymer (nm) heating from 25°C to 121 °C and re-cooling to 25°C | zeta potential/mV | pH before heating | pH after heating |
|---|---|---|---|---|---|
| Example 1 | good | 61/135 | -4.0 (no charge) | 7.1 | 7.2 |
| Example 2 | good | 61/135 | -4.0 (no charge) | 7.1 | 7.2 |
| Example 3 | good | 61/135 | -4.0 (no charge) | 7.1 | 7.2 |
| Example 4 | good | 65/150 | -3.5 (no charge) | 7.1 | 7.2 |
| Example 5 | good | 65/150 | -3.5 (no charge) | 7.1 | 7.2 |
| Example 6 | good | 65/150 | -3.5 (no charge) | 7.1 | 7.2 |
| Example 7 | good | 61/145 | -4.2 (no charge) | 7.1 | 7.1 |
| Example 8 | good | 61/145 | -4.2 (no charge) | 7.1 | 7.2 |
| Example 9 | good | 68/155 | -3.7 (no charge) | 7.1 | 7.1 |
| Example 10 | good | 68/155 | -3.7 (no charge) | 7.1 | 7.2 |
| Example 11 | good | 66/158 | -2.7 (no charge) | 7.1 | 7.2 |
| Example 12 | good | 73/unobservable due to excessive aggregation | -3.1 (no charge) | 7.1 | 7.2 |
| Example 13 | good | 73/unobservable due to excessive aggregation | -3.1 (no charge) | 7.1 | 7.2 |
| Example 14 | good | 79/unobservable due to excessive aggregation | -4.4 (no charge) | 7.1 | 7.2 |
| Example 15 | good | 79/unobservable due to excessive aggregation | -4.4 (no charge) | 7.1 | 7.2 |

[Table 2]

[Table 2]

| | evaluation of liquid film retention time | evaluation of liquid film retention time after 26 hours | sessile droplet contact angle X (°) | water content of coated device (mass%) | change in water content (percentage points) | coefficient of friction | coefficient of friction X | coefficient of friction Y | detection depth of hydrophilic polymer (μm) |
|---|---|---|---|---|---|---|---|---|---|
| Example 1 | A (120 sec) | A (120 sec) | 62 | 55.2 | 0.2 | 0.050 | 0.075 | 0.272 | 1.8 |
| Example 2 | A (120 sec) | A (120 sec) | 75 | 38.2 | 0.2 | 0.133 | 0.151 | 0.265 | 1.6 |
| Example 3 | A (120 sec) | A (120 sec) | 68 | 59.1 | 0.1 | 0.378 | 0.368 | 0.639 | 0.5 |
| Example 4 | A (120 sec) | A (120 sec) | 65 | 55.1 | 0.1 | 0.048 | 0.069 | 0.203 | 2.0 |
| Example 5 | A (120 sec) | A (120 sec) | 76 | 38.1 | 0.1 | 0.109 | 0.121 | 0.193 | 2.2 |
| Example 6 | A (120 sec) | A (120 sec) | 52 | 59.1 | 0.1 | 0.356 | 0.361 | 0.619 | 0.2 |
| Example 7 | A (120 sec) | A (120 sec) | 72 | 46.1 | 0.1 | 0.102 | 0.129 | 0.201 | 1.9 |
| Example 8 | A (120 sec) | A (120 sec) | 74 | 38.1 | 0.1 | 0.131 | 0.141 | 0.250 | 2.0 |
| Example 9 | A (120 sec) | A (120 sec) | 70 | 46.1 | 0.1 | 0.107 | 0.119 | 0.200 | 1.7 |
| Example 10 | A (120 sec) | A (120 sec) | 71 | 38.1 | 0.1 | 0.129 | 0.139 | 0.247 | 1.6 |
| Example 11 | A (120 sec) | A (120 sec) | 73 | 46.1 | 0.1 | 0.100 | 0.117 | 0.198 | 2.5 |
| Example 12 | A (120 sec) | A (120 sec) | 70 | 38.1 | 0.1 | 0.105 | 0.112 | 0.150 | 1.2 |
| Example 13 | A (120 sec) | A (120 sec) | 71 | 46.1 | 0.1 | 0.090 | 0.108 | 0.125 | 1.4 |
| Example 14 | A (120 sec) | A (120 sec) | 72 | 38.1 | 0.1 | 0.112 | 0.128 | 0.168 | 1.4 |
| Example 15 | A (120 sec) | A (120 sec) | 70 | 46.1 | 0.1 | 0.080 | 0.101 | 0.115 | 1.6 |

[Table 3]

[Table 3]

| | amount of lipid adhesion | amount of mucin adhesion (μg/cm²) | tensile elastic modulus of device (MPa) | tensile elastic modulus of coated device (MPa) | rate of change in tensile elastic modulus (%) | size of device (mm) | size of coated device (mm) | rate of change in size (%) |
|---|---|---|---|---|---|---|---|---|
| Example 1 | no adhesion | 5.18 | 0.68 | 0.69 | 1.50 | 14.20 | 14.21 | 0.07 |
| Example 2 | no adhesion | 0.73 | 0.71 | 0.72 | 1.40 | 14.20 | 14.19 | -0.07 |
| Example 3 | no adhesion | 1.65 | 0.26 | 0.27 | 3.80 | 14.20 | 14.21 | 0.07 |
| Example 4 | no adhesion | 5.31 | 0.68 | 0.69 | 1.50 | 14.20 | 14.21 | 0.07 |
| Example 5 | no adhesion | 0.60 | 0.71 | 0.71 | 0.05 | 14.20 | 14.21 | 0.07 |
| Example 6 | no adhesion | 1.71 | 0.26 | 0.27 | 3.80 | 14.20 | 14.19 | -0.07 |
| Example 7 | no adhesion | 0.83 | 0.70 | 0.71 | 1.40 | 14.20 | 14.21 | 0.07 |
| Example 8 | no adhesion | 1.00 | 0.71 | 0.72 | 1.40 | 14.20 | 14.21 | 0.07 |
| Example 9 | no adhesion | 1.67 | 0.70 | 0.71 | 1.40 | 14.20 | 14.19 | -0.07 |
| Example 10 | no adhesion | 1.20 | 0.71 | 0.72 | 1.40 | 14.20 | 14.21 | 0.07 |
| Example 11 | no adhesion | 1.51 | 0.70 | 0.71 | 1.40 | 14.20 | 14.19 | -0.07 |
| Example 12 | no adhesion | 1.10 | 0.71 | 0.72 | 1.40 | 14.20 | 14.21 | 0.07 |
| Example 13 | no adhesion | 1.12 | 0.70 | 0.71 | 1.40 | 14.20 | 14.21 | 0.07 |
| Example 14 | no adhesion | 1.00 | 0.71 | 0.72 | 1.40 | 14.20 | 14.21 | 0.07 |
| Example 15 | no adhesion | 1.10 | 0.70 | 0.71 | 1.40 | 14.20 | 14.21 | 0.07 |

[Comparative example 1]

Except that the device used was a commercially available silicone hydrogel lens, namely, "Acuvue Oasys" (registered trademark), that a 0.2 mass% solution of an acrylic acid / vinylpyrrolidone / N,N-dimethylacrylamide copolymer (polymerization ratio by mole 1/1/2, Mw 550,000, manufactured by Osaka Organic Chemical Industry Ltd.), dissolved in the phosphate buffer solution, was used instead of the solution a, and that the heating in an autoclave was performed at 121 °C for 30 minutes, the same procedure as in Example 1 was carried out. The resulting coated device was evaluated according to the method described above, and results obtained are shown in Tables 4 to 6.

[Comparative example 2]

Except that a 0.1 mass% solution of an acrylic acid / vinylpyrrolidone / N,N-dimethylacrylamide copolymer (polymerization ratio by mole 1/1/2, Mw 330,000, manufactured by Osaka Organic Chemical Industry Ltd.), dissolved in the phosphate buffer solution, was used instead of the solution a, the same procedure as in Comparative example 1 was carried out. The resulting coated device was evaluated according to the method described above, and results obtained are shown in Tables 4 to 6.

[Comparative example 3]

Except that a 0.1 mass% solution of an acrylic acid / N,N-dimethylacrylamide copolymer (polymerization ratio by mole 1/9, Mw 300,000, manufactured by Osaka Organic Chemical Industry Ltd.), dissolved in the phosphate buffer solution, was used instead of the solution a, the same procedure as in Comparative example 1 was carried out. The resulting coated device was evaluated according to the method described above, and results obtained are shown in Tables 4 to 6.

[Comparative example 4]

Except that the device used was a commercially available hydrogel lens, namely, "Medalist One Day Plus" (registered trademark) and that a 0.3 mass% solution of polyethylene glycol (Mw 500,000, manufactured by Wako Pure Chemical Industries, Ltd.), dissolved in the phosphate buffer solution, was used instead of the solution a, the same procedure as in Comparative example 1 was carried out. The resulting coated device was evaluated according to the method described above, and results obtained are shown in Tables 4 to 6.

[Comparative example 5]

Except that a 0.1 mass% solution of an acrylic acid / vinylpyrrolidone copolymer (polymerization ratio by mole 1/4, Mw 590,000, manufactured by Osaka Organic Chemical Industry Ltd.), dissolved in the phosphate buffer solution, was used instead of the solution a, the same procedure as in Comparative example 1 was carried out. The resulting coated device was evaluated according to the method described above, and results obtained are shown in Tables 4 to 6.

[Comparative example 6]

Except that a 0.1 mass% solution of an acrylic acid / vinylpyrrolidone copolymer (polymerization ratio by mole 1/9, Mw 390,000, manufactured by Osaka Organic Chemical Industry Ltd.), dissolved in the phosphate buffer solution, was used instead of the solution a, the same procedure as in Comparative example 1 was carried out. The resulting coated device was evaluated according to the method described above, and results obtained are shown in Tables 4 to 6.

[Comparative example 7]

Except that a 0.2 mass% solution of an acrylic acid / 2-hydroxyethyl methacrylate / N,N-dimethylacrylamide copolymer (polymerization ratio by mole 1/1/2, Mw 430,000, manufactured by Osaka Organic Chemical Industry Ltd.), dissolved in the phosphate buffer solution, was used instead of the solution a, the same procedure as in Comparative example 1 was carried out. The resulting coated device was evaluated according to the method described above, and results obtained are shown in Tables 4 to 6.

[Comparative example 8]

Except that a 0.2 mass% solution of an acrylic acid / 2-hydroxyethyl methacrylate / N,N-dimethylacrylamide copolymer (polymerization ratio by mole 1/1/8, Mw 480,000, manufactured by Osaka Organic Chemical Industry Ltd.), dissolved in the phosphate buffer solution, was used instead of the solution a, the same procedure as in Comparative example 1 was carried out. The resulting coated device was evaluated according to the method described above, and results obtained are shown in Tables 4 to 6.

[Comparative example 9]

Except that a 0.3 mass% solution of polyvinylpyrrolidone (Mw 500,000, manufactured by Osaka Organic Chemical Industry Ltd.), dissolved in the phosphate buffer solution, was used instead of the solution a, the same procedure as in Comparative example 1 was carried out. The resulting coated device was evaluated according to the method described above, and results obtained are shown in Tables 4 to 6.

[Comparative example 10]

Except that a 0.3 mass% solution of poly(N,N-dimethylacrylamide) (Mw 700,000, manufactured by Wako Pure Chemical Industries, Ltd.), dissolved in the phosphate buffer solution, was used instead of the solution a, the same procedure as in Comparative example 1 was carried out. The resulting coated device was evaluated according to the method described above, and results obtained are shown in Tables 4 to 6.

[Comparative example 11]

Except that a phosphate buffer solution was used instead of the solution a and that the heating in an autoclave was performed at 121 °C for 30 minutes, the same procedure as in Example 1 was carried out. The resulting device was evaluated according to the method described above, and results obtained are shown in Tables 4 to 6.

[Comparative example 12]

Except that the device used was a commercially available silicone hydrogel lens, namely, "Acuvue Oasys" (registered trademark), the same procedure as in Comparative example 11 was carried out. The resulting device was evaluated according to the method described above, and results obtained are shown in Tables 4 to 6.

[Comparative example 13]

Except that the device used was a commercially available hydrogel lens, namely, "Medalist One Day Plus" (registered trademark), the same procedure as in Comparative example 11 was carried out. The resulting device was evaluated according to the method described above, and results obtained are shown in Tables 4 to 6.

[Comparative example 14]

Except that the device used was a commercially available hydrogel lens, namely, "Medalist One Day Plus" (registered trademark), the same procedure as in Comparative example 10 was carried out. The resulting coated device was evaluated according to the method described above, and results obtained are shown in Tables 4 to 6.

[Comparative example 15]

Except that a 0.2 mass% solution of the homopolymer prepared in Synthesis example 3, namely, methoxypolyethylene glycol methacrylate (polyethylene glycol chain number 4, Mw 250,000, manufactured by Toray Industries, Inc.), dissolved in the phosphate buffer solution, was used instead of the solution a, the same procedure as in Comparative example 14 was carried out. The resulting coated device was evaluated according to the method described above, and results obtained are shown in Tables 4 to 6.

[Comparative example 16]

Except that a commercially available silicone hydrogel lens, namely, "Ultra One Day" (registered trademark) (manufactured by Bausch & Lomb, kalifilcon A) was used, the same procedure as in Comparative example 15 was carried out. The resulting coated device was evaluated according to the method described above, and results obtained are shown in Tables 4 to 6.

[Comparative example 17]

Except that the device used was a commercially available silicone hydrogel lens, namely, "Acuvue Oasys" (registered trademark), the same procedure as in Comparative example 15 was carried out. The resulting coated device was evaluated according to the method described above, and results obtained are shown in Tables 4 to 6.

[Comparative example 18]

Except that the device used was a commercially available hydrogel lens containing 2-hydroxyethyl methacrylate as the main component, namely, "1day Acuvue" (registered trademark) (manufactured by Johnson & Johnson, etafilcon A), the same procedure as in Comparative example 11 was carried out. The resulting device was evaluated according to the method described above, and results obtained are shown in Tables 4 to 6.

[Comparative example 19]

Except that the device used was a commercially available hydrogel lens containing 2-hydroxyethyl methacrylate copolymerized with MPC monomer (2-methacryloyloxyethyl phosphorylcholine) as the main component, namely, "Proclear 1 Day" (registered trademark) (manufactured by Cooper Vision, omafilcon A), the same procedure as in Comparative example 11 was carried out. The resulting device was evaluated according to the method described above, and results obtained are shown in Tables 4 to 6.

[Comparative example 20]

Except that the device used was a commercially available hydrogel color lens containing 2-hydroxyethyl methacrylate as the main component, namely, "1day Acuvue Define Moist" (registered trademark), the same procedure

as in Comparative example 11 was carried out. The resulting device was evaluated according to the method described above, and results obtained are shown in Tables 4 to 6.

[Comparative example 21]

Except that the device used was a commercially available silicone hydrogel lens containing polyvinylpyrrolidone and silicone as the main components, namely, "1day Acuvue TruEye" (registered trademark), the same procedure as in Comparative example 11 was carried out.

The resulting device was evaluated according to the method described above, and results obtained are shown in Tables 4 to 6.

[Comparative example 22]

Except that the device used was a commercially available silicone hydrogel lens containing silicone as the main component and having a plasma treated lens surface, namely, "Air Optix Aqua" (registered trademark) (manufactured by Alcon Japan Ltd., lotrafilcon A), the same procedure as in Comparative example 11 was carried out. The resulting device was evaluated according to the method described above, and results obtained are shown in Tables 4 to 6.

[Comparative example 23]

Except that a 0.3 mass% solution of the copolymer prepared in Synthesis example 4, namely, a PME-400 / N,N-dimethylacrylamide / 2-hydroxyethyl methacrylate copolymer, dissolved in the phosphate buffer solution, was used instead of the solution a and that the heating in an autoclave was performed at 121°C for 30 minutes, the same procedure as in Example 1 was carried out. The resulting coated device was evaluated according to the method described above, and results obtained are shown in Tables 4 to 6.

[Comparative example 24]

Except that a 0.3 mass% solution of the copolymer prepared in Synthesis example 5, namely, a PME-100 / N,N-dimethylacrylamide / silicone monomer $T_1$ copolymer, dissolved in the phosphate buffer solution, was used instead of the solution a, the same procedure as in Comparative example 23 was carried out. The resulting coated device was evaluated according to the method described above, and results obtained are shown in Tables 4 to 6.

[Comparative example 25]

Except that a 0.3 mass% solution of the copolymer prepared in Synthesis example 6, namely, a PME-100 / N,N-dimethylacrylamide / silicone monomer $T_2$ copolymer, dissolved in the phosphate buffer solution, was used instead of the solution a, the same procedure as in Comparative example 23 was carried out. The resulting coated device was evaluated according to the method described above, and results obtained are shown in Tables 4 to 6.

[Comparative example 26]

Except that a 0.3 mass% solution of the copolymer prepared in Synthesis example 7, namely, a PME-100 / N,N-dimethylacrylamide / hydroxyl group-containing monomethacrylate copolymer, dissolved in the phosphate buffer solution, was used instead of the solution a, the same procedure as in Comparative example 23 was carried out. The resulting coated device was evaluated according to the method described above, and results obtained are shown in Tables 4 to 6.

[Comparative example 27]

Except that a 0.3 mass% solution of the PME-100 / N,N-dimethylacrylamide / hydroxyl group-containing monomethacrylate copolymer prepared in Synthesis example 8, dissolved in the phosphate buffer solution, was used instead of the solution a, the same procedure as in Example 2 was carried out. The resulting coated device was evaluated according to the method described above, and results obtained are shown in Tables 4 to 6.

[Table 4-1]

[Table 4-1]

| | base material | water content of device (mass%) | solution of hydrophilic polymer | hydrophilic polymer polymerization ratio compound a1 /compound a2 /compound a3 |
|---|---|---|---|---|
| Comparative example 1 | silicone hydrogel lens | 38.0 | 0.20 mass% acrylic acid /vinylpyrrolidone /N,N-dimethylacrylamide copolymer | 0/81/0 |
| Comparative example 2 | silicone hydrogel lens | 38.0 | 0.10 mass% acrylic acid /vinylpyrrolidone /N,N-dimethylacrylamide copolymer | 0/81/0 |
| Comparative example 3 | silicone hydrogel lens | 38.0 | 0.10 mass% acrylic acid /N,N-dimethylacrylamide copolymer | 0/93/0 |
| Comparative example 4 | hydrogel lens | 59.0 | 0.30 mass% polyethylene glycol | 0/0/0 |
| Comparative example 5 | silicone hydrogel lens | 38.0 | 0.10 mass% acrylic acid /vinylpyrrolidone copolymer | 0/86/0 |
| Comparative example 6 | silicone hydrogel lens | 38.0 | 0.10 mass% acrylic acid /vinylpyrrolidone copolymer | 0/93/0 |
| Comparative example 7 | silicone hydrogel lens | 38.0 | 0.20 mass% acrylic acid /2-hydroxyethyl methacrylate /N,N-dimethylacrylamide copolymer | 0/50/0 |
| Comparative example 8 | silicone hydrogel lens | 38.0 | 0.20 mass% acrylic acid /2-hydroxyethyl methacrylate /N,N-dimethylacrylamide copolymer | 0/80/0 |
| Comparative example 9 | silicone hydrogel lens | 38.0 | 0.30 mass% polyvinylpyrrolidone | 0/100/0 |
| Comparative example 10 | silicone hydropel lens | 38.0 | 0.30 mass% poly (N,N-dimethylacrylamide) | 0/100/0 |
| Comparative example 11 | silicone hydropel lens | 55.0 | none | none |
| Comparative example 12 | silicone hydropel lens | 38.0 | none | none |
| Comparative example 13 | hydrogel lens | 59.0 | none | none |
| Comparative example 14 | hydrogel lens | 59.0 | 0.30 mass% poly (N,N-dimethylacrylamide) | 0/100/0 |
| Comparative example 15 | hydrogel lens | 59.0 | 0.20 mass% methoxypolyethylene glycol methacrylate homopolymer (ethylene oxide repeating unit number 4) | 100/0/0 |
| Comparative example 16 | silicone hydropel lens | 55.0 | 0.20 mass% methoxypolyethylene glycol methacrylate homopolymer (ethvlene oxide repeating unit number 4) | 100/0/0 |

(continued)

| | base material | water content of device (mass%) | solution of hydrophilic polymer | hydrophilic polymer polymerization ratio compound a1 /compound a2 /compound a3 |
|---|---|---|---|---|
| Comparative example 17 | silicone hydropel lens | 38.0 | 0.20 mass% methoxypolyethylene glycol methacrylate homopolymer (ethvlene oxide repeating unit number 4) | 100/0/0 |
| Comparative example 18 | hydrogel lens | 58.0 | none | none |
| Comparative example 19 | hydrogel lens | 60.0 | none | none |
| Comparative example 20 | hydrogel lens | 58.0 | none | none |
| Comparative example 21 | silicone hydrogel lens | 46.0 | none | none |
| Comparative example 22 | silicone hydropel lens | 33.0 | none | none |
| Comparative example 23 | silicone hydrogel lens | 55.0 | 0.30 mass% polyalkylene glycol monomethacrylate with side chain terminal methyl /N,N-dimethylacrylamide /2-hydroxyethyl methacrylate copolymer | 35/56/0 |
| Comparative example 24 | silicone hydrogel lens | 55.0 | 0.30 mass% polyalkylene glycol monomethacrylate with side chain terminal methyl /N,N-dimethylacrylamide /copolymer of chemical formula (II) | 62/34/0 |
| Comparative example 25 | silicone hydrogel lens | 55.0 | 0.30 mass% polyalkylene glycol monomethacrylate with side chain terminal methyl /N,N-dimethylacrylamide /copolymer of chemical formula (**III**) | 63/34/0 |
| Comparative example 26 | silicone hydrogel lens | 55.0 | 0.30 mass% polyalkylene glycol monomethacrylate with side chain terminal methyl /N,N-dimethylacrylamide /glycerol methacrylate copolymer | 13/25/62 |
| Comparative example 27 | silicone hydrogel lens | 38.0 | 0.30 mass% polyalkylene glycol monomethacrylate with side chain terminal methyl /N,N-dimethylacrylamide /glycerol methacrylate copolymer | 24/69/7 |

[Table 4-2]

[Table 4-2]

| | filterability of hydrophilic polymer | average particle diameter of hydrophilic polymer (nm) heating from 25°C to 121 °C and re-cooling to 25°C | zeta potential/mV | pH before heating | pH after heating |
|---|---|---|---|---|---|
| Comparative example 1 | good | 62/63 | -21.0 | 7.0 | 7.0 |
| Comparative example 2 | good | 62/63 | -21.0 | 7.0 | 7.1 |
| Comparative example 3 | good | 61/64 | -21.1 | 6.9 | 7.0 |
| Comparative example 4 | good | unmeasured | -1.8 (no charge) | 7.0 | 7.0 |
| Comparative example 5 | good | 63/65 | -21.3 | 6.9 | 7.0 |
| Comparative example 6 | good | 63/65 | -21.3 | 6.9 | 7.0 |
| Comparative example 7 | good | 61/64 | -20.7 | 6.8 | 6.9 |
| Comparative example 8 | good | 61/64 | -20.7 | 7.0 | 7.1 |
| Comparative example 9 | good | unmeasured | -2.8 (no charge) | 7.0 | 7.1 |
| Comparative example 10 | good | unmeasured | -2.5 (no charge) | 7.1 | 7.2 |
| Comparative example 11 | none | none | none | 7.0 | 7.1 |
| Comparative example 12 | none | none | none | 7.0 | 7.2 |
| Comparative example 13 | none | none | none | 7.0 | 7.1 |
| Comparative example 14 | good | unmeasured | -2.5 (no charge) | 7.1 | 7.2 |
| Comparative example 15 | good | unmeasured | -1.9 (no charge) | 7.1 | 7.2 |
| Comparative example 16 | good | unmeasured | -1.9 (no charge) | 7.1 | 7.2 |
| Comparative example 17 | good | unmeasured | -1.9 (no charge) | 7.1 | 7.2 |
| Comparative example 18 | none | none | none | 7.0 | 7.0 |
| Comparative example 19 | none | none | none | 7.0 | 7.0 |
| Comparative example 20 | none | none | none | 7.0 | 7.0 |
| Comparative example 21 | none | none | none | 7.0 | 7.0 |
| Comparative example 22 | none | none | none | 7.0 | 7.0 |

(continued)

| | filterability of hydrophilic polymer | average particle diameter of hydrophilic polymer (nm) heating from 25°C to 121 °C and re-cooling to 25°C | zeta potential/mV | pH before heating | pH after heating |
|---|---|---|---|---|---|
| Comparative example 23 | good | 55/55 | -4.3 (no charge) | 7.0 | 7.0 |
| Comparative example 24 | unmeasurable (incomplete dissolution of polymer) | unmeasured | -4.0 (no charge) | 7.1 | 7.2 |
| Comparative example 25 | unmeasurable (incomplete dissolution of polymer) | unmeasured | -3.8 (no charge) | 7.0 | 7.1 |
| Comparative example 26 | poor | 60/125 | -3.1 (no charge) | 7.0 | 7.1 |
| Comparative example 27 | poor | 61/110 | -4.3 (no charge) | 7.1 | 7.2 |

[Table 5]

[Table 5]

| | evaluation of liquid film retention time | evaluation of liquid film retention time after 26 hours | sessile droplet contact angle X (°) | water content of coated device (mass%) | change in water content (percentage points) | coefficient of friction | coefficient of friction X | coefficient of friction Y | detection depth of hydrophilic polymer (μm) |
|---|---|---|---|---|---|---|---|---|---|
| Comparative example 1 | D (**1** sec) | D (**1** sec) | 102 | 38.0 | 0 | 0.250 | 0.357 | 0.360 | 0 |
| Comparative example 2 | D (**1** sec) | D (**1** sec) | 103 | 38.0 | 0 | 0.248 | 0.351 | 0.358 | 0 |
| Comparative example 3 | D (1 sec) | D (1 sec) | 101 | 38.0 | 0 | 0.251 | 0.350 | 0.351 | 0 |
| Comparative example 4 | A (90 sec) | D (3 sec) | 78 | 59.0 | 0 | 0.120 | 0.350 | 0.355 | 0 |
| Comparative example 5 | D (1 sec) | D (1 sec) | 107 | 38.0 | 0 | 0.254 | 0.348 | 0.348 | 0 |
| Comparative example 6 | D (**1** sec) | D (1 sec) | 103 | 38.0 | 0 | 0.261 | 0.340 | 0.344 | 0 |
| Comparative example 7 | D (**1** sec) | D (**1** sec) | 102 | 38.0 | 0 | 0.262 | 0.349 | 0.351 | 0 |
| Comparative example 8 | D (1 sec) | D (**1** sec) | 105 | 38.0 | 0 | 0.259 | 0.353 | 0.355 | 0 |
| Comparative example 9 | A (120 sec) | D (3 sec) | 99 | 38.0 | 0 | 0.123 | 0.326 | 0.348 | 0 |
| Comparative example 10 | A (120 sec) | D (3 sec) | 100 | 38.0 | 0 | 0.110 | 0.351 | 0.329 | 0 |
| Comparative example **11** | A (120 sec) | A (120 sec) | 107 | none | none | 0.157 | 0.421 | 0.475 | none |
| Comparative example 12 | D (7 sec) | D (3 sec) | 105 | none | none | 0.340 | 0.359 | 0.467 | none |
| Comparative example 13 | D (9 sec) | D (3 sec) | 83 | none | none | 0.350 | 0.804 | 0.754 | none |
| Comparative example 14 | A (120 sec) | D (3 sec) | 77 | 59.0 | 0 | 0.124 | 0.350 | 0.351 | 0 |
| Comparative example 15 | A (120 sec) | D (2 sec) | 77 | 59.0 | 0 | 0.351 | 0.360 | 0.365 | 0 |
| Comparative example 16 | A (120 sec) | A (120 sec) | 100 | 55.0 | 0 | 0.355 | 0.370 | 0.375 | 0 |
| Comparative example 17 | A (120 sec) | A (90 sec) | 103 | 38.0 | 0 | 0.341 | 0.350 | 0.360 | 0 |
| Comparative example 18 | D (3 sec) | D (1 sec) | 80 | none | none | 0.434 | 0.455 | 0.461 | none |
| Comparative example 19 | D (3 sec) | D (**1** sec) | 84 | none | none | 0.323 | 0.351 | 0.352 | none |
| Comparative example 20 | D (3 sec) | D (**1** sec) | 81 | none | none | 0.450 | 0.461 | 0.463 | none |
| Comparative example 21 | D (3 sec) | D (**1** sec) | 107 | none | none | 0.360 | 0.370 | 0.379 | none |
| Comparative example 22 | D (2 sec) | D (1 sec) | 103 | none | none | 0.420 | 0.431 | 0.433 | none |

(continued)

| | evaluation of liquid film retention time | evaluation of liquid film retention time after 26 hours | sessile droplet contact angle X (°) | water content of coated device (mass%) | change in water content (percentage points) | coefficient of friction | coefficient of friction X | coefficient of friction Y | detection depth of hydrophilic polymer (μm) |
|---|---|---|---|---|---|---|---|---|---|
| Comparative example 23 | A (120 sec) | A (120 sec) | 106 | 55.0 | 0 | 0.165 | 0.450 | 0.480 | 0 |
| Comparative example 24 | A (120 sec) | A (120 sec) | 105 | 55.0 | 0 | 0.160 | 0.461 | 0.475 | 0 |
| Comparative example 25 | A (120 sec) | A (120 sec) | 107 | 55.0 | 0 | 0.171 | 0.478 | 0.489 | 0 |
| Comparative example 26 | A (120 sec) | A (120 sec) | 105 | 55.0 | 0 | 0.175 | 0.456 | 0.469 | 0 |
| Comparative example 27 | D (8 sec) | D (5 sec) | 105 | 38.0 | 0 | 0.342 | 0.348 | 0.350 | 0 |

[Table 6]

[Table 6]

| | amount of lipid adhesion | amount of mucin adhesion (μg/cm2) | **tensile** elastic modulus of device (MPa) | tensile elastic modulus of coated device (MPa) | rate of change in tensile elastic modulus (%) | size of device (mm) | size of coated device (mm) | rate of change in size (%) |
|---|---|---|---|---|---|---|---|---|
| Comparative example 1 | adhered on 20% of total area | 1.27 | 0.71 | 0.72 | 1.40 | 14.20 | 14.21 | (%) 0.07 |
| Comparative example 2 | adhered on 20% of total area | 1.21 | 0.71 | 0.71 | 0.56 | 14.20 | 14.20 | 0.00 |
| Comparative example 3 | adhered on 20% of total area | 1.99 | 0.71 | 0.70 | -1.40 | 14.20 | 14.21 | 0.07 |
| Comparative example 4 | no adhesion | 1.75 | 0.26 | 0.26 | 0.00 | 14.20 | 14.21 | 0.07 |
| Comparative example 5 | adhered an 20% of total area | 1.30 | 0.71 | 0.71 | 0.56 | 14.20 | 14.20 | 0.00 |
| Comparative example 6 | adhered on 20% of total area | 1.19 | 0.71 | 0.71 | 0.00 | 14.20 | 14.20 | 0.00 |
| Comparative example 7 | adhered on 20% of total area | 1.29 | 0.71 | 0.71 | 0.00 | 14.20 | 14.19 | -0.07 |
| Comparative example 8 | adhered on 20% of total area | 1.31 | 0.71 | 0.71 | 0.80 | 14.20 | 14.21 | 0.07 |
| Comparative example 9 | adhered on 20% of total area | 1.28 | 0.71 | 0.71 | 0.90 | 14.20 | 14.21 | 0.07 |
| Comparative example 10 | adhered on 20% of total area | 1.25 | 0.71 | 0.71 | 0.70 | 14.20 | 14.21 | 0.07 |
| Comparative example 11 | **adhered** on 5% of total area | 5.34 | 0.68 | 0.68 | 0.00 | 14.20 | 14.20 | 0.00 |
| Comparative example 12 | **adhered** on 20% of total area | 1.24 | 0.71 | 0.71 | 0.00 | 14.20 | 14.20 | 0.00 |
| Comparative example 13 | no adhesion | 1.70 | 0.26 | 0.26 | 0.00 | 14.20 | 14.20 | 0.00 |
| Comparative example 14 | no adhesion | 1.80 | 0.26 | 0.26 | 0.00 | 14.20 | 14.21 | 0.07 |

(continued)

| | amount of lipid adhesion | amount of mucin adhesion ($\mu g/cm^2$) | **tensile** elastic modulus of device (MPa) | tensile elastic modulus of coated device (MPa) | rate of change in tensile elastic modulus (%) | size of device (mm) | size of coated device (mm) | rate of change in size (%) |
|---|---|---|---|---|---|---|---|---|
| Comparative example 15 | no adhesion | 1.71 | 0.26 | 0.26 | 0.00 | 14.20 | 14.21 | 0.07 |
| Comparative example 16 | adhered on 5% of total area | 5.35 | 0.68 | 0.68 | 0.00 | 14.20 | 14.21 | 0.07 |
| Comparative example 17 | adhered on 20% of total area | 1.30 | 0.71 | 0.72 | 1.40 | 14.20 | 14.21 | 0.07 |
| Comparative example 18 | no adhesion | 2.10 | 0.30 | none | none | 14.20 | none | none |
| Comparative example 19 | no adhesion | 3.07 | 0.39 | none | none | 14.20 | none | none |
| Comparative example 20 | no adhesion | 2.50 | 0.30 | none | none | 14.20 | none | none |
| Comparative example 21 | adhered on 20% of total area | 1.84 | 0.70 | none | none | 14.20 | none | none |
| Comparative example 22 | adhered on 20% of total area | 2.59 | 1.10 | none | none | 14.20 | none | none |
| Comparative example 23 | adhered on 5% of total area | 5.25 | 0.68 | 0.68 | 0.00 | 14.20 | 14.20 | 0.00 |
| Comparative example 24 | adhered on 5% of total area | 5.31 | 0.68 | 0.69 | 1.50 | 14.20 | 14.20 | 0.00 |
| Comparative example 25 | adhered on 5% of total area | 5.29 | 0.68 | 0.68 | 0.00 | 14.20 | 14.20 | 0.00 |
| Comparative example 26 | adhered on 5% of total area | 5.35 | 0.68 | 0.69 | 1.50 | 14.20 | 14.20 | 0.00 |
| Comparative example 27 | adhered on 20% of total area | 1.28 | 0.71 | 0.71 | 0.00 | 14.20 | 14.20 | 0.00 |

From a comparison of results obtained in Examples and those obtained in Comparative examples, it is clear that the present invention serves to impart to a device high lipid adhesion resistance and mucin adhesion resistance, in addition to sufficient hydrophilicity and lubricity.

Furthermore, if focusing on the change in the coefficient of friction between before and after immersion in a phosphate buffer solution for 26 hours, that is, the difference between the above coefficient of friction and the coefficient of friction X, a system that is smaller in the change can be said to have a device surface that is coated with a hydrophilic polymer with higher adsorption force, superior durability, and higher lubricity. Results obtained in Examples 1 to 15 prove this feature. Meanwhile, if focusing on rubbing resistance, a system that is smaller in the coefficient of friction Y than devices devoid of a hydrophilic polymer can be said to have a device surface that is coated with a hydrophilic polymer with

particularly high adsorption force. The results obtained in Examples 1, 2, 4, and 5, and Examples 7 to 15, which are focused on silicone hydrogel type devices, prove this feature.

## Claims

1. A coated device comprising a device and a hydrophilic polymer that coats the surface of the device, wherein the hydrophilic polymer includes a hydrophilic polymer A, the hydrophilic polymer A containing, as monomer units, a compound a1 as represented by the general formula (I) given below, a compound a2 having an amide group, and a compound a3 having a plurality of hydroxyl groups, the polymerization ratio among the compound a1, compound a2, and compound a3 being such that the compound a1 accounts for 1 to 96 mass%, the compound a2 for 1 to 96 mass%, and the compound a3 for 8 to 61 mass%,

[chemical compound 1]

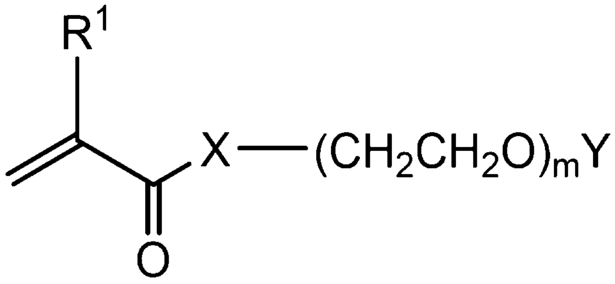

· · · (I)

wherein, in the general formula (I), $R^1$ represents a hydrogen atom or a methyl group; X represents an oxygen atom or $NR^2$; $R^2$ represents a hydrogen atom or an alkyl group; m represents an integer of 1 to 30; and Y represents a hydrogen atom or an alkyl group.

2. A coated device as set forth in claim 1, wherein the compound a3 is glycerol (meth)acrylate.

3. A coated device as set forth in either claim 1 or 2, wherein the change in the average particle diameter of the hydrophilic polymer A is irreversible when heating it and then cooling it back.

4. A coated device as set forth in either claim 1 or 2, wherein the detection depth of the hydrophilic polymer A is 10.00 μm or less.

5. A coated device as set forth in either claim 1 or 2, wherein the hydrophilic polymer A that coats the surface of the device is present nonuniformly in a frozen state.

6. A coated device as set forth in either claim 1 or 2, wherein the device is a medical device.

7. A coated device as set forth in claim 6, wherein the device contains one material selected from the group consisting of hydrogel material, silicone hydrogel material, low-water-content soft material, and low-water-content hard material.

8. A coated device as set forth in claim 7, wherein the hydrogel material is one selected from the group consisting of tefilcon, tetrafilcon, helfilcon, mafilcon, polymacon, hioxifilcon, alfafilcon, omafilcon, nelfilcon, nesofilcon, hilafilcon, acofilcon, deltafilcon, etafilcon, focofilcon, ocufilcon, phemfilcon, methafilcon, and vilfilcon.

9. A coated device as set forth in claim 7, wherein the silicone hydrogel material is one selected from the group consisting of lotrafilcon, galyfilcon, narafilcon, senofilcon, comfilcon, enfilcon, balafilcon, efrofilcon, fanfilcon, somofilcon, samfilcon, olifilcon, asmofilcon, formofilcon, stenfilcon, abafilcon, mangofilcon, riofilcon, sifilcon, larafilcon, kalifilcon, and delefilcon.

10. A coated device as set forth in claim 7, wherein either the low-water-content soft material or the low-water-content hard material is one selected from the group consisting of polysulfone, polystyrene, polymethyl methacrylate, polyurethane, and polyamide.

**11.** A coated device as set forth in claim 6, wherein the device is one selected from the group consisting of ophthalmic lenses, skin covering materials, wound dressing materials, skin protective materials, skin drug carriers, infusion tubes, gas transport tubes, liquid drainage tubes, blood circuits, coating tubes, catheters, stents, sheaths, biosensor chips, artificial heart and lung components, and endoscope covering materials.

**12.** A production method for a coated device as set forth in either claim 1 or 2 comprising a contact step (A) in which a device is placed in a container, followed by bringing the device into contact with a solution a of a hydrophilic polymer A and a heating step (C) in which the container is heated, wherein the hydrophilic polymer A contains, as monomer units, a compound a1 as represented by the general formula (I) given below, a compound a2 having an amide group, and a compound a3 having a plurality of hydroxyl groups, the polymerization ratio among the compound a1, compound a2, and compound a3 being such that the compound a1 accounts for 1 to 96 mass%, the compound a2 for 1 to 96 mass%, and the compound a3 for 8 to 61 mass%,

[chemical compound 2]

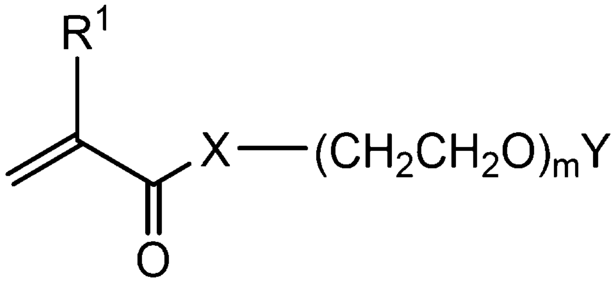

・・・(I)

wherein, in the general formula (I), $R^1$ represents a hydrogen atom or a methyl group; X represents an oxygen atom or $NR^2$; $R^2$ represents a hydrogen atom or an alkyl group; m represents an integer of 1 to 30; and Y represents a hydrogen atom or an alkyl group, and the solution a after the heating step having a pH in the range of 6.1 to 8.0.

**13.** A production method for a coated device as set forth in claim 12 further comprising a sealing step (B) for sealing the container that houses the device.

**14.** A production method for a coated device as set forth in claim 12, wherein the heating step also serves for sterilization of the device.

**INTERNATIONAL SEARCH REPORT**

International application No.
**PCT/JP2024/016431**

**A. CLASSIFICATION OF SUBJECT MATTER**

***A61L 27/34***(2006.01)i; ***A61L 15/24***(2006.01)i; ***A61L 15/26***(2006.01)i; ***A61L 27/16***(2006.01)i; ***A61L 27/18***(2006.01)i; ***A61L 27/52***(2006.01)i; ***A61L 31/06***(2006.01)i; ***A61L 31/10***(2006.01)i; ***A61L 31/14***(2006.01)i; ***A61L 33/06***(2006.01)i
FI: A61L27/34; A61L15/24; A61L15/26; A61L27/16; A61L27/18; A61L27/52; A61L31/06; A61L31/10; A61L31/14 300; A61L33/06 200; A61L33/06 300

According to International Patent Classification (IPC) or to both national classification and IPC

**B. FIELDS SEARCHED**

Minimum documentation searched (classification system followed by classification symbols)

A61L27/34; A61L15/24; A61L15/26; A61L27/16; A61L27/18; A61L27/52; A61L31/06; A61L31/10; A61L31/14; A61L33/06

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | WANG, Chunyan et al. Synthesis and Performance of Novel Hydrogels Coatings for Implantable Glucose Sensors. Biomacromolecules. September 2008, 561-567, DOI:10.1021/bm701102y<br>entire text, all drawings | 1-3, 5-7, 10-14 |
| A | | 4 |
| X | TRZEBINSKI, Jakub et al. Hydrogel Membrane Improves Batch-to-Batch Reproducibility of an Enzymatic Glucose Biosensor. Electroanalysis. 2011, 23, 2789-2795, DOI:10.1002/elan.201100286<br>entire text, all drawings | 1-3, 5-7, 10-14 |
| A | | 4 |
| Y | WO 2022/185836 A1 (TORAY INDUSTRIES, INC.) 09 September 2022 (2022-09-09)<br>claims, paragraphs [0007], [0016]-[0031], [0037]-[0045], [0071], examples | 1-14 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **18 June 2024** | **02 July 2024** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (July 2022)

**INTERNATIONAL SEARCH REPORT**

International application No.
**PCT/JP2024/016431**

**C. DOCUMENTS CONSIDERED TO BE RELEVANT**

| Category* | Citation of document, with indication, where appropriate, of the relevant passages | Relevant to claim No. |
|---|---|---|
| Y | US 2007/0264503 A1 (LAI, Yuchin) 15 November 2007 (2007-11-15) claims, paragraphs [0008], [0033], [0039]-[0044], examples | 1-14 |
| A | JP 6954490 B1 (TORAY INDUSTRIES, INC.) 27 October 2021 (2021-10-27) entire text, all drawings | 1-14 |
| A | WO 2022/185837 A1 (TORAY INDUSTRIES, INC.) 09 September 2022 (2022-09-09) entire text, all drawings | 1-14 |
| A | WO 2020/235275 A1 (TORAY INDUSTRIES, INC.) 26 November 2020 (2020-11-26) entire text, all drawings | 1-14 |
| P, A | WO 2024/043096 A1 (TORAY INDUSTRIES, INC.) 29 February 2024 (2024-02-29) entire text, all drawings | 1-14 |

Form PCT/ISA/210 (second sheet) (July 2022)

**INTERNATIONAL SEARCH REPORT**
**Information on patent family members**

International application No.
**PCT/JP2024/016431**

| Patent document cited in search report | Publication date (day/month/year) | Patent family member(s) | Publication date (day/month/year) |
|---|---|---|---|
| WO 2022/185836 A1 | 09 September 2022 | EP 4285948 A1<br>claims, paragraphs [0016]-[0029], [0035]-[0042], [0067], examples<br>CN 116897056 A<br>KR 10-2023-0151511 A | |
| US 2007/0264503 A1 | 15 November 2007 | US 2007/0264509 A1<br>US 2008/0197324 A1 | |
| JP 6954490 B1 | 27 October 2021 | US 2023/0057266 A1<br>entire text, all drawings<br>EP 4066867 A1<br>KR 10-2022-0098405 A<br>CN 114828904 A | |
| WO 2022/185837 A1 | 09 September 2022 | EP 4285949 A1<br>entire text, all drawings<br>CN 116897057 A<br>KR 10-2023-0151510 A | |
| WO 2020/235275 A1 | 26 November 2020 | US 2022/0184908 A1<br>entire text, all drawings<br>EP 3944003 A1<br>CN 113785236 A | |
| WO 2024/043096 A1 | 29 February 2024 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2017018425 A
- WO 2015119256 A
- JP 5154231 B
- WO 2013024799 A
- WO 2013024800 A
- JP 2014533381 W
- JP 6954490 B